# EUROPEAN PATENT APPLICATION

(11) **EP 4 293 017 A1**
(43) Date of publication of application: **20.12.2023**
(21) Application number: 22753053.2
(22) Date of filing: 01.02.2022
(51) Int. Cl.: C07D 333/66, C07D 333/68, A61K 31/381, A61P 31/14, A61P 31/16

(54) **2-ACETAMIDO-6-HYDROXY-BENZOTHIOPHENE DERIVATIVES WITH ANTIVIRAL ACTIVITY**

(30) Priority: 12.02.2021 RU 2021103521
(71) Applicant: Otcpharm JSC, Moscow, 123112 (RU)
(72) Inventor: MAKAROV, Vadim Albertovich, Moscow, 115035 (RU); ZARUBAEV, Vladimir Viktorovich, Saint Petersburg, Petrodvorets, 198504 (RU)
(74) Representative: Boskovic, Davor
(86) International application number: PCT/RU2022/050030
(87) International publication number: WO 2022/173329

(57) **Abstract**

The invention relates to a novel derivative of 2-acetamido-6-hydroxy-benzothiophene of general formula I, or a pharmaceutically acceptable salt thereof, which has antiviral activity and can be used for treating viral diseases caused by respiratory RNA viruses, influenza viruses or coronaviruses. The primary use of the claimed compounds is for treating diseases where the virus is an influenza virus or the coronavirus SARS-CoV-2 and the disease is influenza A or COVID-19.

## Description

The invention relates to 2-acetamido-6-hydroxy-benzothiophene derivatives of general formula (I) and pharmaceutically acceptable salts thereof as novel biologically active compounds exhibiting activity against various viruses, mainly against the influenza virus and SARS-CoV-2 coronavirus, and to a method for preparation and use thereof as antiviral drugs.

The problem of finding new antiviral agents is accounted for by the significant spread of viral infections in humans and animals. This is due to the decreasing immune protection in the human population, as well as the widespread development of viral resistance to the available drugs in the market. The problem of the rapidly developing resistance is caused by the fact that many of the known used antiviral drugs are generally derivatives of one class of compounds, as well as by the lack of efficacy and often high toxicity of drugs.

Influenza is an acute infectious disease of the respiratory tract caused by an RNA-containing virus, which has a high epidemiological and clinical significance with a high frequency of complications, especially among people at risk. The viruses of influenza and other acute respiratory viral infections (ARVI) cause massive outbreaks of diseases that turn into epidemics every year. From 27 to 41 million cases of these diseases are registered annually, in particular, from 5 to 15% of the Russian population evey year fall ill with influenza. Influenza and ARVI remain practically uncontrolled diseases due to the high variability of the antigenic structure of circulating influenza viruses and the heterogeneity of ARVI pathogens. Furthermore, influenza and other ARVI viruses are able to change their properties and pathogenicity. A recent example of such changes is the causative agent of H1N1pdm09 influenza circulating in the epidemic season of 2009-2010 and called "swine flu".

Antivirals for the treatment of influenza represent a very limited group of drugs, and most of them are known to be resistant to viruses. Due to the genome organization features (lacking mechanism for correcting replication errors) and a short life cycle, the influenza virus has a high mutation rate. As a result, the antigenic structure of the virus is highly susceptible to change under the selective pressure of the host's immune system. Moreover, the virus perceives the used chemotherapy as a selection factor, whereby the formation of resistant strains also occurs. These two processes lead to the emergence of virus variants that are able to avoid both the activity of neutralizing antibodies, whereby they elude the body's immune response, and to overcome the effect of chemotherapy drugs aimed at a certain stage of virus reproduction. Furthermore, each type of virus has the own mechanism of adaptation to a chemical drug [Ison M.G. Antivirals and resistance: influenzavirus, Current Opinion in Virology, 2011, 563].

There are known neuraminidase inhibitors registered in Russia: Oseltamivir (Tamiflu) and Zanamivir (Relenza), as well as those used in the USA: Peramivir (Rapiacta) and Laninamivir (Inavir), which act at the stage of budding of newly synthesized influenza virions from the cell membrane, blocking the cleavage of viral progeny particles from the cell surface [Ison M.G. Clinical use of approved influenza antivirals: therapy and prophylaxis. // Influenza Other Respir. Viruses. 2013, 7 Suppl. 1, 7-13]. Furthermore, viral neuraminidase inhibitors prevent the access of virions to the target cells by blocking the neuraminidase cleavage of mucopolysaccharides in the mucus of the upper respiratory tract. The practice of using neuraminidase inhibitors in the treatment of influenza has shown that the high efficacy of this group of drugs is limited by the early stage of the disease.

There are also known anti-influenza drugs of a different mechanism of action, for example, Remantadine (α-methyl-1-adamantylmethylamine hydrochloride) and Amantadine (1-aminoadamantane) [Davies, W.L.; Grunert, R.R.; Haff, R.F.; McGahen, J.W.; Neumayer, E.M; Paulshock, M.; Watts, J.C; Wood, T.R.; Hermann, E.C; Hoffmann, C.E. Antiviral Activity of 1-Adamantanamine (Amantadine) // Science. 1964, V. 144, P. 862]. These compounds block the M2 protein of the influenza virus, thereby preventing the process of hemagglutinin cleavage, the fusion of the membranes of the virus and the lysosomal vacuole, and the virus "undressing" process. [Scholtissek C., Quack G., Klenk H.D., Webster R.G. // Antiviral Res. 1998, V. 37, P. 83-95]. The mechanism of action of these drugs is well studied. [Cady S.D., Schmidt-Rohr K., Wang J., Soto C.S., DeGrado W.F., Hong M.H. Structure of the amantadine binding site of influenza M2 proton channels in lipid bilayers, Nature. 2010, 463, 689]. Adamantane drugs are much cheaper and easier in manufacture than commercially available neuraminidase inhibitors and, therefore, more accessible for the treatment and prevention of influenza in the population. However, at present, as a result of the widespread use of adamantane drugs, their antiviral properties against influenza A viruses have been significantly lost. Nevertheless, framework compounds remain attractive as a basis for the design of antiviral drugs. Thus, we know deutiforin (2-(1'-aminoethyl)bicyclo[2.2.1]heptane), which is one of the most interesting drugs based on the natural bicyclic framework compounds (bornanes) [Patent RU 2448692 C2 of 27.04.2012], as well as recently developed camphecin (1,7,7-trimethylbicyclo[2.2.1]heptan-2-ylidene-aminoethanol) [RF Patent No. 2530554 of 22.04.2013].

Baloxaviramarboxyl (Xofluza), an inhibitor of the endonuclease activity of the influenza virus polymerase complex, has been registered in the United States (Yang T. Baloxavir Marboxil: The First Cap-dependent Endonuclease inhibitor for the treatment of influenza. Ann. Pharmacother. 2019, 53, 7, 754-759).

In Russia and some other countries, Arbidol (umifenovir) is used as a drug to control influenza by blocking the fusogenic activity of viral hemagglutinin and thus preventing the fusion of viral and cell membranes.

[Blaising, J., Polyak, S.J., Pécheur, E-I., Arbidolas a broad-spectrumantiviral: anupdate. Antiviral Research (2014), doi: http://dx.doi.org/10.1016/j.antivira1.2014.04.006]. In addition to a direct antiviral activity, it has interferonogenic properties and thus can be used both for the therapy and for the prevention of influenza infection.

A disadvantage of the drugs based on adamantane derivatives is their low antiviral activity caused by the resistance of the vast majority of influenza strains to this drug. There are also reported influenza virus variants that are resistant to neuraminidase inhibitors, in particular, to Tamiflu as the most common drug. This resistance is based on amino acid substitutions at or near the NA catalytic site. For example, the H274Y mutation provides oseltamivir resistance of the viruses carrying the N1 subtype neuraminidase, while the E119V and R292K mutations determine the resistance of the N2 subtype [McKimm-Breschkin J. L. Influenza neuraminidase inhibitors: antiviral action and mechanisms of resistance. Influenza Other Resp. Viruses. 2013, 7, Suppl. 1, 25]. In general, according to the Center for Disease Control (CDC), the prevalence of oseltamivir-resistant viruses in the United States in the period preceding the pandemic remained at a low level and did not exceed 1%, and in 2008-2009 the level of resistance to oseltamivir increased to 12%, after which a gradual decrease started in the number of resistant strains. Within the H1N1 subtype, however, the level of oseltamivir resistance has reached almost 100% in all regions of the Earth [Okomo-Adhiambo M., Fry A. M., Su S. et al. Oseltamivir-resistant influenza A (H1N1) pdm09 viruses, United States, 2013-14. Emerg. Infect. Dis. 2015, 21, 136].

Virus strains resistant to baloxavir have also been isolated from patients treated with this new drug (Takashita E, Abe T, Morita H, Nagata S, et al. Influenza A (H1N1) pdm09 virus exhibiting reduced susceptibility to baloxavir due to a PA E23K substitution detected from a child without baloxavir treatment. Antiviral Res. 2020, 180, 104828).

Thus, influenza viruses demonstrate an ability to develop resistance to direct antiviral drugs, regardless of the mechanism of their activity. One way to overcome such resistance is the simultaneous use of several drugs that target different viral targets. In this case, the probability of selecting virus variants that are resistant to two or more drugs is greatly reduced. Despite this, the formation of such double mutants resistant to both adamantane drugs and neuraminidase inhibitors was demonstrated by a number of studies [Sheu T.G., Fry A.M., Garten R.J. Dual Resistance to Adamantanes and Oseltamivir Among Seasonal Influenza A (H1N1) Viruses: 2008-2010. Journal of Infectious Diseases 2011, 203, 13].

The nature of RNA viruses, influenza and coronavirus suggests the effective use of systemic administration of interferon drugs (viferon, intron, reaferon, etc.) for basic nonspecific therapy of infections caused by these viruses, taking into account the asthenization caused thereby. The efficacy of topical application of interferon solutions is doubtful and can be considered in the presence of local symptoms (rhinitis, pharyngitis, etc.). The use of interferon inducers (amixin, cycloferon, neovir, etc.) suggests the formation of secondary immunosuppression in 10-14 days, which can lead to re-infection in the ongoing epidemic period. The basic antiviral therapy drugs include the targeted drugs that affect the key viral processes: viral genome replication (remdesivir, triazavirin, favipiravir, and ribavirin, which is the strongest but also the most toxic drug in this group), viral neuraminidase activity (oseltamivir, zanamivir, peramivir), proteolysis of viral polyproteins by means of virus-specific proteases (lopinavir, ritonavir, nelfinavir) [Yamamoto N., Matsuyama S., Hoshino T. Nelfinavir inhibits replication of severe acute respiratory syndrome coronavirus 2 in vitro. BioRxiv 2020.04.06.026476 [Preprint]. 2020: biorxiv.org/content/10.1101/2020.04.06.026476v1]. Anti-replicative activity was traced for isoprinosine, a purine derivative, which exhibits its activity against influenza A and B viruses.

Thus, the biology of influenza viruses and coronaviruses inevitably determines the emergence of their new pandemic strains having unpredictable time of occurrence, genome variability, and antigenic properties. Therefore, pandemics of new respiratory infections will always begin in the absence of specific immune prophylaxis and therapy for these infections. The latter predetermines the need for early research and development of pathogenetic drugs and methods for the prevention/treatment of respiratory viral infections based on the biological features of coronaviruses and influenza A viruses.

For the most complete and effective control of influenza infection, an urgent task is the constant development and implementation of new antiviral drugs that are chemically unrelated to those already used in the clinical practice and aimed at alternative targets in the virus life cycle.

The objective of the present invention is to find new pharmacologically active compounds against viral infections, which would have low toxicity and would not cause side effects in warm-blooded living organisms, primarily against influenza and coronavirus, including the strains, which are resistant to the currently existing drugs.

The technical effect comprises an increasing efficacy of antiviral drugs, in particular, against the resistant strains of viruses, a lower toxicity of antiviral agents, and a wider selection of antiviral drugs.

This problem is solved, and the technical effect is achieved by synthesizing 2-acetamido-6-hydroxy-benzothiophene derivatives corresponding to general structural formula I: 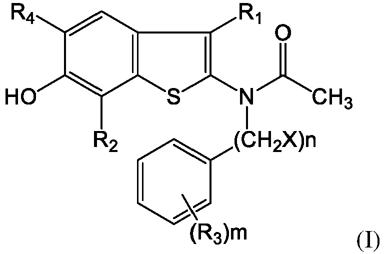 where
R¹ is CN, COOH, COOAlk, CONHAlk, CON(Alk)₂, CONH₂;
R² is H, Hal, CH₂(NAlk)₂;
R³ is Hal, Alk, OAlk, CF₃, or two adjacent R³ groups form -CH=CH-CH=CH- group;
R⁴ is H or Hal;
n = 1-4; m = 1-3;
X is hydrogen or methyl;
Hal is fluorine, chlorine or bromine;
each Alk is a linear or branched alkyl group having 1 to 4 carbon atoms, or
in N(Alk)₂ group, two Alk groups together with the nitrogen atom to which they are attached form a -(CH₂)₃₋₅-group, in which one of said -CH₂- groups may be substituted by a nitrogen atom or a -N-CH₃ group.

The preferential compounds of general formula (I) are those, where R¹ is COOAlk or CONHAlk, in (CH₂X)ₙ group X is H, n is 1.

The examples of such compounds comprise:
a compound according to formula (I), where R¹ is COOMe, R² is H, Cl or CH₂(NAlk)₂., and 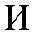 n = 1; or
a compound according to formula (I), where R¹ is CONHMe, R² is H, Cl or CH₂(NAlk)₂, and n = 1; or
a compound according to formula (I), where R¹ is CN, R² is H, Cl or CH₂(NAlk)₂, and n = 1.

The most important distinctive feature of the presented compounds and their key characteristic is the presence of both a benzyl (substituted benzyl) and an acetyl substituent at the nitrogen atom in the second position of benzothiophene.

The invention also relates to pharmaceutically acceptable salts of the compounds of formula I.

The term of "pharmaceutically acceptable salts of a compound of formula (I)" means salts of an inorganic or organic acid or base which have the desired pharmacological activity of the parent compound. These salts can be obtained *in situ* during the synthesis, isolation or purification of the compound of formula (I) or prepared specially.

The pharmaceutically acceptable acid salts are characterized in that they comprise the therapeutically active non-toxic acid addition salt forms, which are able to form the compounds of formula I. These acid addition salts can be obtained by treating the base compound represented by general formula I with suitable acids, for example inorganic acids: hydrohalic acid, in particular hydrochloric acid, hydrobromic acid, sulfuric acid, nitric acid or phosphoric acid; organic acids, for example: acetic acid, hydroxyacetic acid, propionic acid, lactic acid, pyruvic acid, oxalic acid, malonic acid, maleic acid or fumaric acid, malic acid, tartaric acid or citric acid.

In case the compound has a free COOH group, the compound can form alkali metal salts, for example the sodium salt.

The compounds can be obtained and used in crystalline form.

The compounds of general formula I and pharmaceutically acceptable salts thereof have antiviral activity and can be used in the treatment of diseases caused by viral infection, in particular caused by RNA-containing respiratory viruses or coronaviruses. The compounds of general formula I and pharmaceutically acceptable salts thereof can be used for the treatment or prophylaxis of influenza, for example influenza A, and/or diseases caused by the SARS-CoV-2 coronavirus, for example, COVID-19.

The compounds of general formula I and pharmaceutically acceptable salts thereof have been studied with respect to pathogenic viruses and can be used to obtain drugs based thereon for the treatment or prevention of viral diseases caused by RNA-containing respiratory viruses or coronaviruses, in particular, for example, caused by influenza virus or coronavirus SARS-CoV-2, such as influenza or COVID-19.

The objective of the present invention is also solved, and the technical effect is achieved by creating a pharmaceutical composition comprising an effective amount of the compound of formula I or pharmaceutically acceptable salt thereof as an active ingredient and at least one pharmaceutically acceptable excipient.

"Pharmaceutically acceptable excipients" mean diluents, excipients and/or pharmaceutical carriers used in the pharmaceutical field.

Pharmaceutical carriers mean the carriers which are used in the pharmaceutical field in the preparation of a drug. When preparing a composition as a dosage form for oral administration, binders, lubricants, disintegrants, solvents, diluents, stabilizers, suspending agents, colorless agents, flavoring agents are used; injection forms comprise antiseptic agents, solubilizers, stabilizers; and topical forms comprise bases, diluents, lubricating agents, antiseptic agents.

The pharmaceutical composition may be used as a drug in a tablet, capsule or topical form.

The pharmaceutical composition can be obtained by mixing the active ingredient in an effective amount and the appropriate excipient.

The problem of the present invention is also solved, and the technical effect is achieved by using derivatives of 2-acetamido-6-hydroxy-benzothiophene of general formula I and pharmaceutically acceptable salts thereof, possibly in crystalline form, or a pharmaceutical composition based thereon for the preparation of drugs for the prevention and treatment of diseases mediated viral infection caused by viruses such as RNA-containing respiratory viruses or coronaviruses, in particular, for example, caused by the influenza virus or the SARS-CoV-2 coronavirus. In particular, a disease may be influenza or COVID-19.

The problem of the present invention is also solved, and the technical effect is achieved creating a method for preventing or treating a disease mediated by a viral infection, comprising the administration or application to the subject of a 2-acetamido-6-hydroxy-benzothiophene derivative of general structural formula I, pharmaceutically acceptable salt thereof or a pharmaceutical composition based thereon in an effective amount. The viral infection may be an infection caused by RNA-containing respiratory viruses or coronaviruses, in particular, for example, caused by the influenza virus or the SARS-CoV-2 coronavirus.

The method of treatment using a compound of the invention, a pharmaceutical composition or a drug based thereon is also effective against strains resistant to currently existing drugs.

The *in vitro* and *in vivo* experiments showed a high activity exhibited by 2-acetamide-6-hydroxy-benzothiophene compounds of formula I against influenza virus and SARS-CoV-2 coronavirus.

See below the examples demonstrating the preparation of the compounds according to the invention. These examples comprise the particular embodiments of the present invention, but do not limit the present invention.

### Examples of implementation of the invention and realization of the purpose

### Materials and methods

All reagents and solvents were purchased from commercial suppliers (AlfaAesar, Acros, Himmed) and used without further purification.

The ¹H and ¹³C NMR spectra were recorded with Bruker AC-300 (operating frequency 300 MHz, ¹H) and Bruker AC-200 (operating frequency 50 MHz, ¹³C) spectrometers in DMSO-*d₆* or CDCl₃ as solvents. Chemical shifts are given in ppm on the *δ* scale relative to Me₄Si.

The mass spectra of the compounds were recorded with a Finnigan MAT INCO 50 quadrupole mass spectrometer (electron impact, ionization energy 70 eV) with the sample directly introduced into the ion source.

The high performance liquid chromatography with tandem mass spectroscopy was performed with an Agilent 1290 Infinity system comprising an Agilent 6460 triple quadrupole mass spectrometric detector, a binary pump, a mobile phase degasser, a column thermostat, and an autosampler. The chromatographic separation was carried out with an Agilent Eclipse Plus C18 RRHD column (2.1 × 50 mm, 1.8 µm) at 40°C. The volume of the injected sample was 2 µl. The mobile phase comprised eluent A: 0.1% formic acid/water and eluent B: 0.1% formic acid and 85% acetonitrile in water. The gradient was 0.0-3.0 min: 60% B (separation), 3.0-4.0 min: 60 → 97% B (rinsing and regeneration), 4.0-6.0 min: 97% B, 6.0-6.1 min: 97 → 60% B, 6.1-9.0 min: 60% B. The mobile phase flow rate was 0.4 ml/min. The mass spectrometric detection was performed under positive ionization. The optimal parameters comprised capillary voltage 3.5 kV, drying nitrogen gas, temperature 350°C, and flow rate 12 l/min. The purity of all final compounds was over 95%.

The elemental composition of the synthesized compounds was determined with a CHNS Elemental Analyzer EURO EA.

The melting points were determined with an Electrothermal 9001 without correction.

The reaction progress and substance identity were controlled by thin layer chromatography using Merck KGaA TLC Silicagel 60 F₂₅₄ plates. Spots were detected by irradiation with UV light.

The mixtures of compounds were separated out by column chromatography (SiO₂, 0.006-0.2 mm (70-230 mesh)).

The yields are given for the purified products without optimization.

### General synthesis method 1.

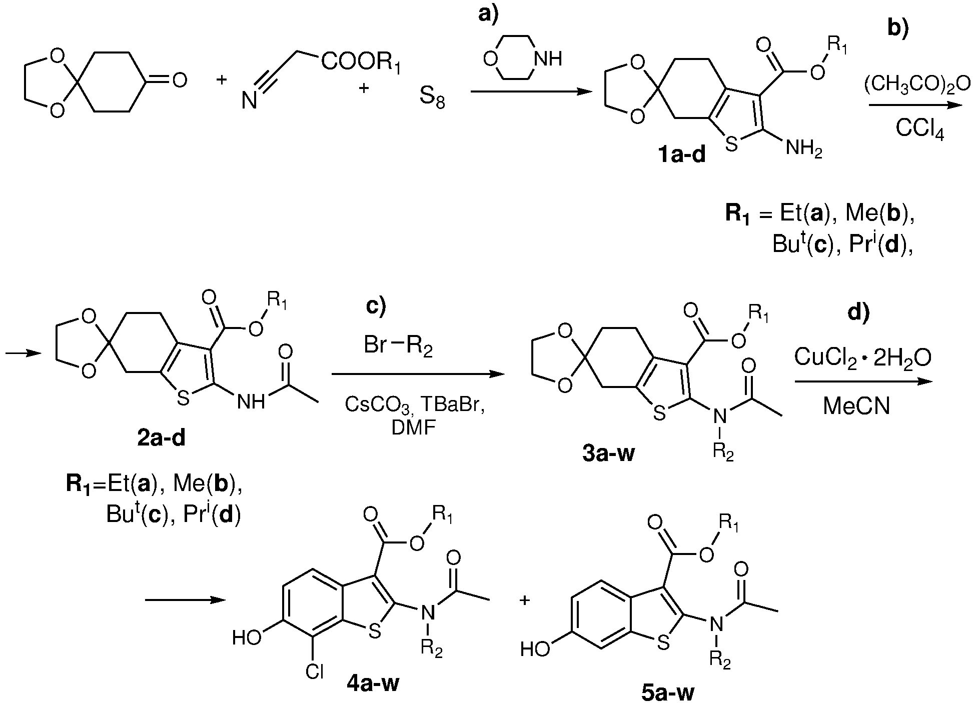

| | -R₁ | -R₂ |
|---|---|---|
| **3-5a** | Et | 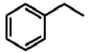 |
| **3-5b** | Me | 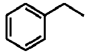 |
| **3-5c** | Bu^{t} | 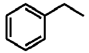 |
| **3-5d** | Prⁱ | 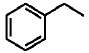 |
| **3-5e** | Et | 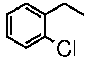 |
| **3-5f** | Et | 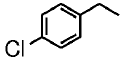 |
| **3-5g** | Et | 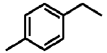 |
| **3-5h** | Me | 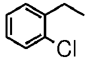 |
| **3-5i** | Me | 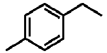 |
| **3-5j** | Me | 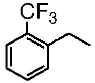 |
| **3-5k** | Me | 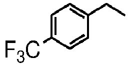 |
| **3-5l** | Me | 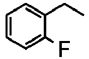 |
| **3-5m** | Me | 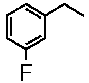 |
| **3-5n** | Me | 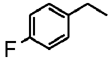 |
| **3-5o** | Me | 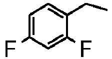 |
| **3-5p** | Me | 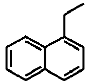 |
| **3-5q** | Me | 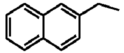 |
| **3-5r** | Me | 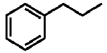 |
| **3-5s** | Me | 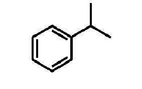 |
| **3-5t** | Me | 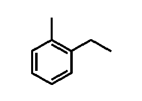 |
| **3-5u** | Me | 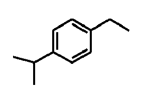 |
| **3-5v** | Me | 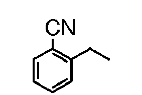 |
| **3-5w** | Me | 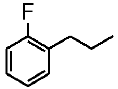 |

### a) Preparation of 2-amino-4,7-dihydro-5H-spiro[1-benzothiophene-6,2'-[1,3]dioxolane]-3-carboxylate derivatives la-d.

A mixture of 1.4-dioxospiro[4.5]decan-8-one (40 mmol), ethyl cyanoacetate (40 mmol) or methyl cyanoacetate (40 mmol) or isopropyl cyanoacetate (40 mmol) or tert-butyl cyanoacetate (40 mmol), sulfur (40 mmol) and morpholine (40 mmol) in 40 ml of the appropriate alcohol is stirred for 4 hours at 45°C. Volatiles are removed under vacuum and the residue is dissolved in 150 ml of ethyl acetate. The solution is transferred to a separating funnel, washed with water to pH 6.5, dried over sodium sulfate, and the ethyl acetate is evaporated.

*Ethyl 2-amino-4,7-dihydro--5H-spiro[1-benzothiophene-6,2'-[1*,*3]dioxolane]-3-carboxylate* **1a.** Yield 92%, oil. Mass (EI), *m*/*z* (*Iᵣₑₗₐₜ*.(%)): 283.3444 [M]⁺ (57). C₁₃H₁₇NO₄S.

*Methyl 2-amino-4,7-dihydro-5H-spiro[1-benzothiophene-6,2'-[1*,*3]dioxolane)-3-carboxylate* **1b.** Yield 84%. Mp. 101-3°C. Mass (EI), *m*/*z* (*Iᵣₑₗₐₜ*.(%)): 269.3178 [M]⁺ (62). C₁₂H₁₅NO₄S.

*Isopropyl 2-amino-4,7-dihydro-5H-spiro[-benzothiophene-6,2'-[1*,*3]dioxolane]-3-carboxylate* **1c**. Yield 93%, oil. Mass (EI), *m*/*z* (*Iᵣₑₗₐₜ*.(%)): 297.371 [M]⁺ (52).C₁₄H₁₉NO₄S.

*Tert-butyl 2-amino-4,7-dihydro-5H-spiro]1-benzothiophene-6,2'-[1*,*3)dioxolane)-3-carboxylate* **1d**. Yield 90%, oil. Mass (EI), *m*/*z* (*Iᵣₑₗₐₜ*.(%)): 311.3976 [M]⁺ (49). C₁₅H₂₁NO₄S.

### b) Preparation of 2-(acetylamino)-4,7-dihydro-5H-spiro[1-benzothiophene-6,2'-[1,3]dioxolane]-3-carboxylate derivatives 2a-d.

Acetic anhydride (60 mmol) is added to a suspension of compound **1** (30 mmol) in 80 ml of carbon tetrachloride and refluxed for 2 hours. The solution is evaporated under vacuum, water is added to the resulting residue, and the precipitate is filtered off, washed with water, methanol or ethanol, and diethyl ether.

*Ethyl 2-(acetylamino)-4,7-dihydro-5H-spiro[1-benzothiophene-6,2'-[1*,*3]dioxolane)-3-carboxylate* **2a**. Yield: 80%. Mp. 128-130°C. Mass (EI), *m*/*z* (*Iᵣₑₗₐₜ*.(%)): 325.3811 [M]⁺ (82). C₁₅H₁₉NO₅S.

*Methyl 2-(acetylamino)-4,7-dihydro-5H-spiro] 1-benzothiophene-6,2'-[1*,*3]dioxolane]-3-carboxylate* **2b**. Yield: 89%. Mp. 143-147°C. Mass (EI), *m*/*z* (*Iᵣₑₗₐₜ*.(%)): 311.3545 [M]⁺ (87). C₁₄H₁₇NO₅S.

*Propyl 2-(acetylamino)-4,7-dihydro-5H-spiro[1-benzothiophene-6,2'-[1*,*3]dioxolane]-3-carboxylate* **2c**. Yield:60%, oil. Mass (EI), *m*/*z* (*Iᵣₑₗₐₜ*.(%)): 339.4077 [M]⁺ (78). C₁₆H₂₁NO₅S.

*Tert-butyl 2-(acetylamino)-4,7-dihydro-5H-spiro[1-benzothiophene-6,2'-[1*,*3]dioxolane]-3-carboxylate* **2d**. Yield: 59%. Mp. 129-133°C. Mass (EI), *m*/*z* (*Iᵣₑₗₐₜ*.(%)): 353.4343 [M]⁺ (68). C₁₆H₂₁NO₅S.

### c) Preparation of 2-[acetyl(benzyl)amino]-4,7-dihydro-5H-spiro[1-benzothiophene-6,2'-[1,3]dioxolane]-3-carboxylate derivatives 3a-w.

Cesium carbonate (15 mmol), tetrabutylammonium bromide (15 mmol) and the corresponding benzyl bromide (15 mmol) are added to a solution of compound **2** (10 mmol) in 40 ml of dry dimethylformamide. The reaction mixture is stirred at room temperature for 2 to 4 hours (TLC monitoring in hexane:ethyl acetate 7:3). 150 ml of ethyl acetate and 200 ml of water are added. The organic layer is washed again with 100 ml of water and dried with sodium sulfate for 4 hours, and ethyl acetate is evaporated to prepare compounds **3,** most of which are oils. Purification is carried out by column chromatography, using chloroform as eluent.

*Ethyl 2-[acetyl(benzyl)amino]-4,7-dihydro-5H-spiro[1-benzothiophene-6,2'-[1,3]dioxolane]-3-carboxylate* **3a**. Yield: 70%. Mp. 66-70°C. Mass (EI), *m*/*z* (*Iᵣₑₗₐₜ.*(%)): 415.5036 [M]⁺ (45). C₂₂H₂₅NO₅S.

*Methyl 2-[acetyl(benzyl)amino]-4,7-dihydro-5H-spiro[1-benzothiophene-6,2'-[1,3]dioxolane]-3-carboxylate* **3b.** Yield: 88%. Mp. 64-66°C. Mass (EI), *m*/*z* (*Iᵣₑₗₐₜ*.(%)): 401.4771 [M]⁺ (34). C₂₁H₂₃NO₅S.

*Isopropyl 2-[acetyl(benzyl)amino]-4,7-dihydro-5H-spiro[1-benzothiophene-6,2'-[1,3]dioxolane]-3-carboxylate* **3c**. Yield: 64%, oil. Mass (EI), *m*/*z* (*Iᵣₑₗₐₜ*.(%)): 429.5302 [M]⁺ (32). C₂₃H₂₇NO₅S.

*Tert-butyl 2-[acetyl(benzyl)amino]-4,7-dihydro-5H-spiro]1-benzothiophene-6,2'-[1,3]dioxolane]-3-carboxylate* **3d**. Yield: 88%, oil. Mass (EI), *m*/*z* (*Iᵣₑₗₐₜ.*(*%*)): 443.5568 [M]⁺ (34). C₂₄H₂₉NO₅S.

*Ethyl 2-[acetyl(2-chlorobenzyl)amino]-4,7-dihydro-5H-spiro[1-benzothiophene-6,2'-[1,3]dioxolane]-3-carboxylate* **3e**. Yield: 82%, oil. Mass (EI), *m*/*z* (*Iᵣₑₗₐₜ*.(%)): 449.9484 [M]⁺ (24). C₂₂H₂₄ClNO₅S.

*Ethyl 2-[acetyl(4-chlorobenzyl)amino]-4,7-dihydro-5H-spiro[1-benzothiophene-6,2'-[1,3]dioxolane]-3-carboxylate* **3f**. Yield: 93%, oil. Mass (EI), *m*/*z* (*Iᵣₑₗₐₜ*.(%)): 449.9484 [M]⁺ (28). C₂₂H₂₄ClNO₅S.

*Ethyl 2-]acetyl]4-methylbenzyl)amino]-4,7-dihydro-5H-spiro[1-benzothiophene-6,2'-[1,3]dioxolane]-3-carboxylate* **3g**. Yield: 81%, oil. Mass (EI), *m*/*z* (*Iᵣₑₗₐₜ.*(%)): 429.5302 [M]⁺ (35). C₂₃H₂₇NO₅S.

*Methyl 2-[acetyl(2-chlorobenzyl)amino]-4,7-dihydro-5H-spiro[1-benzothiophene-6,2'-[1,3]dioxolane]-3-carboxylate* **3h**. Yield: 80%, oil. Mass (EI), *m*/*z* (*Iᵣₑₗₐₜ*.(%)): 435.9218 [M]⁺ (38). C₂₁H₂₂ClNO₅S.

*Methyl 2-[acetyl(4-methylbenzyl)amino]-4,7-dihydro-5H-spiro[1-benzothiophene-6,2'-[1,3]dioxolane]-3-carboxylate* **3i**. Yield: 87%, oil. Mass (EI), *m*/*z* (*Iᵣₑₗₐₜ*.(%)): 415.503 [M]⁺ (22). C₂₂H₂₅NO₅S.

*Methyl 2-[acetyl(2-trifluoromethylbenzyl)amino]-4,7-dihydro-5H-spiro[1-benzothiophene-6,2'-[1,3]dioxolane]-3-carboxylate* **3j**. Yield: 88%, oil. Mass (EI), *m*/*z* (*Iᵣₑₗₐₜ*.(%)): 469.475 [M]⁺ (21). C₂₂H₂₂F₃NO₅S.

*Methyl 2-[acetyl(4-trifluoromethylbenzyl)amino]-4,7-dihydro-5H-spiro[1-benzothiophene-6,2'-[1,3]dioxolane]-3-carboxylate* **3k**. Yield: 85%, oil. Mass (EI), *m*/*z* (*Iᵣₑₗₐₜ*.(%)): 469.475 [M]⁺ (24). C₂₂H₂₂F₃NO₅S.

*Methyl 2-[acetyl(2-fluorobenzyl)amino]-4,7-dihydro-5H-spiro[1-benzothiophene-6,2'-[1,3]dioxolane]-3-carboxylate* **3I**. Yield: 93%, oil. Mass (EI), *m*/*z* (*Iᵣₑₗₐₜ.*(%)): 419.4675 [M]⁺ (29). C₂₁H₂₂FNO₅S.

*Methyl 2-[acetyl(3-fluorobenzyl)amino]-4,7-dihydro-5H-spiro[1-benzothiophene-6,2*'-*[1,3]dioxolane]-3-carboxylate* **3m**. Yield: 90%, oil. Mass (EI), *m*/*z* (*Iᵣₑₗₐₜ*.(%)): 419.4675 [M]⁺ (28). C₂₁H₂₂FNO₅S.

*Methyl 2-[acetyl(4-fluorobenzyl)amino]-4,7-dihydro-5H-spiro[1-benzothiophene-6,2'-[1,3]dioxolane]-3-carboxylate* **3n**. Yield: 86%, oil. Mass (EI), *m*/*z* (*Iᵣₑₗₐₜ*.(%)): 419.4675 [M]⁺ (25). C₂₁H₂₂FNO₅S.

*Methyl 2-[acetyl( 2,4-difluorobenzyl)amino]-4,7-dihydro-5H-spiro[1-benzothiophene-6,2'-[1,3]dioxolane]-3-carboxylate* **3o**. Yield: 87%, oil. Mass (EI), *m*/*z* (*Iᵣₑₗₐₜ*.(%)): 437.458 [M]⁺ (17). C₂₁H₂₁F₂NO₅S.

*Methyl 2-[acetyl(1-naphthylmethyl)amino)-4,7-dihydro-5H-spiro[1-benzothiophene-6,2'-[1,3]dioxolane]-3-carboxylate* **3p**. Yield: 92%, oil. Mass (EI), *m*/*z* (*Iᵣₑₗₐₜ*.(%)): 451.5357 [M]⁺ (19). C₂₅H₂₅NO₅S.

*Methyl 2-[acetyl(2-naphthylmethyl)amino]-4,7-dihydro-5H-spiro[1-benzothiophene-6,2'-[1,3]dioxolane]-3-carboxylate* **3q**. Yield: 93%, oil. Mass (EI), *m*/*z* (*Iᵣₑₗₐₜ*.(%)): 451.5357 [M]⁺ (15). C₂₅H₂₅NO₅S.

*Methyl 2-[acetyl(2-phenylethyl)amino]-4,7-dihydro-5H-spiro[1-benzothiophene-6,2'-[1,3]dioxolane]-3-carboxylate* **3r**. Yield: 75%, oil. Mass (EI), *m*/*z* (*Iᵣₑₗₐₜ*.(%)): 415.5036 [M]⁺ (23). C₂₂H₂₅NO₅S.

*Methyl 2-[acetyl(1-phenylethyl)amino]-4,7-dihydro-5H-spiro[1-benzothiophene-6,2'-[1,3]dioxolane]-3-carboxylate* **3s**. Yield: 87%, oil. Mass (EI), *m*/*z* (*Iᵣₑₗₐₜ.*(*%*)): 415.5036 [M]⁺ (22). C₂₂H₂₅NO₅S.

*Methyl 2-[acetyl(2-methylbenzyl)amino]-4,7-dihydro-5H-spiro[1-benzothiophene-6,2'-[1,3]dioxolane]-3-carboxylate* **3t.** Yield: 84%, oil. Mass (EI), *m*/*z* (*Iᵣₑₗₐₜ*.(%)): 415.503 [M]⁺ (20). C₂₂H₂₅NO₅S.

*Methyl 2-[acetyl(4-isopropylbenzyl)amino]-4,7-dihydro-5H-spiro[1-benzothiophene-6,2'-[1,3]dioxolane]-3-carboxylate* **3u**. Yield: 91%, oil. Mass (EI), *m*/*z* (*Iᵣₑₗₐₜ*.(%)): 443.5568 [M]⁺ (18). C₂₄H₂₉NO₅S.

*Methyl 2-[acetyl(2-cyanobenzyl)amino]-4,7-dihydro-5H-spiro[1-benzothiophene-6,2'-[1,3]dioxolane]-3-carboxylate* **3v**. Yield: 89%, oil. Mass (EI), *m*/*z* (*Iᵣₑₗₐₜ*.(%)): 428.5024 [M]⁺ (22). C₂₂H₂₄N₂O₅S.

*Methyl 2-[acetyl(2-fluorophenylethyl)amino]-4,7-dihydro-5H-spiro[1-benzothiophene-6,2'-[1,3]dioxolane]-3-carboxylate* **3w**. Yield: 69%, oil. Mass (EI), *m*/*z* (*Iᵣₑₗₐₜ*.(%)): 435.51 [M]⁺ (23). C₂₂H₂₄FNO₅S.

d) Preparation of methyl-2-[acetyl(benzyl)amino]-7-chloro-6-hydroxy-1-benzothiophene-3-carboxylate derivatives **4a-w** 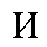 methyl 2-[acetyl(benzyl)amino]-6-hydroxy-1-benzothiophene-3-carboxylate derivatives **5a-w.**

Copper(II) chloride dihydrate (20 mmol) is added to a solution of compound **3** (10 mmol) in 70 ml of acetonitrile and stirred at room temperature for 2 to 4 hours (TLC control in dichloromethane:ethyl acetate 19:1). The reaction mixture is treated with 80 ml of 2 N HCl solution, 50 ml of water and 200 ml of ethyl acetate. The organic layer is washed with water until pH 6-7. Ethyl acetate is removed under vacuum. The resulting oil is a mixture of reaction products **4a-w** and **5a-w,** which are isolated individually by column chromatography (dichloromethane:ethyl acetate 19:1 system is used).

### Example 1.

*Ethyl 2-[acetyl(benzyl)amino]-7-chloro-6-hydroxy-1-benzothiophene-3-carboxylate **4a.***

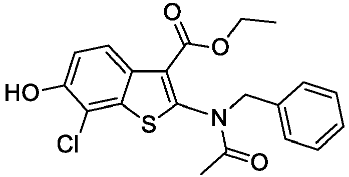

Yield: 20%.

Mp. 151-155°C. Mass (EI), *m*/*z* (*Iᵣₑₗₐₜ*.(%)): 403.88 [M]⁺ (40). C₂₀H₁₈ClNO₄S. ¹H NMR (300 MHz, DMSO-d₆) δ 1.27 (t, *J* = 7.1 Hz, 3H, CH₃CH₂), 1.85 - 2.11 (br. s, 3H, CH₃CO), 4.20 (br. q, *J* = 7.1Hz, 2H, CH₃CH₂), 4.87 (br. s, 2H, NCH₂Ph), 7.07 - 7.43 (m, 6H, C₆H₅, H5), 8.07 (d, *J* = 8.9 Hz, 1H, H4), 10.54 (s, 1H, OH).

### Example 2.

*Ethyl 2-[acetyl(benzyl)amino]-6-hydroxy-1-benzothiophene-3-carboxylate **5a.***

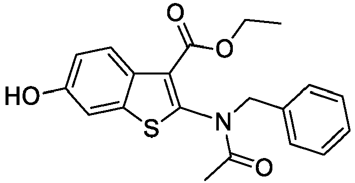

Yield: 39%. Mp. 146-148°C. Mass (EI), *m*/*z* (*Iᵣₑₗₐₜ.*(*%*)): 369.4352 [M]⁺ (38). C₂₀H₁₉NO₄S. ¹H NMR (300 MHz, DMSO-d₆) δ 1.25 (t, *J* = 7.1 Hz, 3H, CH₃CH₂), 1.95 (s, 3H, CH₃CO), 4.17 (br. s, 2H, CH₃CH₂), 4.56 (d, *J* = 13.0 Hz, 1H, NCHPh), 5.08 (d, *J* = 13.0 Hz, 1H, NCHPh), 6.98 (dd, *J* = 8.9, 2.3 Hz, 1H, H5), 7.11 - 7.38 (m, 6H, C₆H₅, H7), 8.10 (d, *J* = 8.9 Hz, 1H, H4), 9.86 (s, 1H, OH).

### Example 3.

*Methyl 2-[acetyl(benzyl)amino]-7-chloro-6-hydroxy-1-benzothiophene-3-carboxylate **4b.***

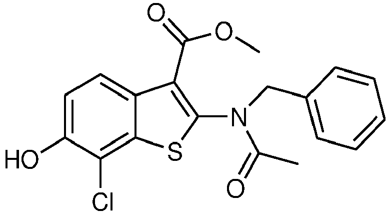

Yield: 22%. Mp. 218-222°C. Mass (EI), *m*/*z* (*Iᵣₑₗₐₜ*.(%)): 389.8534 [M]⁺ (45). C₁₉H₁₆ClNO₄S. ¹H NMR (300 MHz, DMSO-d₆) δ 1.94 (s, 3H, CH₃CO), 3.72 (s, 3H, OCH₃), 4.72 (br. s, 1H, NCH₂), 4.99 (br. s, 1H, NCH₂), 7.05 - 7.30 (m, 6H), 8.06 (d, *J* = 8.9 Hz, 1H, H4), 10.70 (s, 1H, OH).

### Example 4.

*Methyl 2-[acetyl(benzyl)amino]-6-hydroxy-1-benzothiophene-3-carboxylate **5b.***

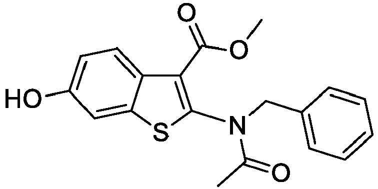

Yield: 35%. Mp. 165-167°C. Mass (EI), *m*/*z* (*Iᵣₑₗₐₜ*.(%)): 355.4086 [M]⁺ (50). C₁₉H₁₇NO₄S. ¹H NMR (300 MHz, DMSO-d₆) δ 1.94 (s, 3H, CH₃CO), 3.72 (s, 3H, OCH₃), 4.64 (br. s, 1H, NCH₂), 4.99 (br. s, 1H, NCH₂), 6.97 (dd, *J* = 9.0, 2.4 Hz, 1H, H5), 7.13 - 7.32 (m, 6H), 8.06 (d, *J* = 8.9 Hz, 1H, H4), 9.86 (s, 1H, OH).

### Example 5.

*Tert*-*butyl 2-[acetyl(benzyl)amino]-7-chloro-6-hydroxy-1-benzothiophene-3-carboxylate **4c**.*

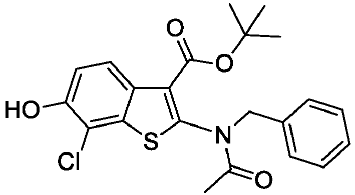

Yield: 19%. Mp. 200-205°C. Mass (EI), *m*/*z* (*Iᵣₑₗₐₜ*.(%)): 431.9331 [M]⁺ (31). C₂₂H₂₂ClNO₄S. ¹H NMR (200 MHz, DMSO-d₆) δ 1.50 (s, 9H, (CH₃)₃C), 1.94 (s, 3H, CH₃CO), 4.31 (d, *J* = 14.8 Hz, 1H), 5.37 (d, *J* = 14.8 Hz, 1H), 7.05 - 7.40 (m, 6H), 8.12 (d, *J* = 8.9 Hz, 1H, H4), 10.8 (s, *J* = 2.4 Hz, 1H, OH).

### Example 6.

*Tert-butyl 2-[acetyl(benzyl)amino]-6-hydroxy-1-benzothiophene-3-carboxylate **5c**.*

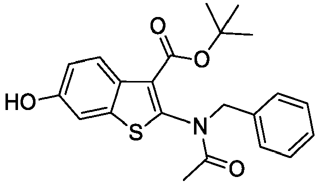

Yield: 10%. Mp. 185-190°C. Mass (EI), *m*/*z* (*Iᵣₑₗₐₜ.*(%)): 397.4884 [M]⁺ (34). C₂₂H₂₃NO₄S. ¹H NMR (200 MHz, DMSO-d₆) δ 1.51 (s, 9H, (CH₃)₃C), 1.94 (s, 3H, CH₃CO), 4.28 (d, *J* = 14.8 Hz, 1H), 5.37 (d, *J* = 14.8 Hz, 1H), 6.97 (dd, *J* = 9.0, 2.3 Hz, 1H, H5), 7.11 - 7.42 (m, 6H), 8.13 (d, *J* = 8.9 Hz, 1H, H4), 9.87 (s, *J* = 2.4 Hz, 1H, OH).

### Example 7.

*Isopropyl 2-[acetyl(benzyl)amino]-7-chloro-6-hydroxy-1-benzothiophene-3-carboxylate **4d.***

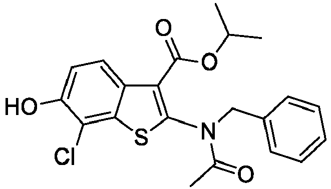

Yield: 20%. Mp. 164-168°C. Mass (EI), *m*/*z* (*Iᵣₑₗₐₜ*.(%)): 417.9065 [M]⁺ (35). C₂₁H₂₀ClNO₄S. ¹H NMR (300 MHz, DMSO-d₆) δ 1.27 (d, *J* = 6.2 Hz, 6H, (CH₃)₂CH), 1.96 (s, 3H, CH₃CO), 4.43 (d, *J* = 15.0 Hz, 1HNCH₂), 5.13 (hept, *J* = 6.2 Hz, CHO), 5.27 (d, *J* = 15.0 Hz, 1H, NCH₂), 7.44 - 7.08 (m, 6H), 8.11 (d, *J* = 8.8 Hz, 1H, H4), 10.72 (s, 1H, OH).

### Example 8.

*Isopropyl 2-[acetyl(benzyl)amino]-6-hydroxy-1-benzothiophene-3-carboxylate **5d.***

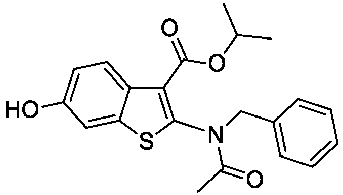

Yield: 11%. Mp. 126-130°C. Mass (EI), *m*/*z* (*Iᵣₑₗₐₜ*.(%)): 383.4618 [M]⁺ (44). C₂₁H₂₁NO₄S. ¹H NMR (300 MHz, DMSO-d₆) δ 1.28 (d, *J* = 6.3 Hz, 6H, (CH₃)₂CH), 2.01 (s, 3H, CH₃CO), 4.37 (br. s, 1H, NCH₂), 5.11 (hept, *J* = 6.2 Hz, 1H, CHO), 5.28 (br. s, 1H, NCH₂), 6.98 (dd, *J* = 8.9, 2.4 Hz, 1H, H5), 7.10 - 7.43 (m, 6H), 8.13 (d, *J* = 8.9 Hz, 1H, H4), 9.85 (s, 1H, OH).

### Example 9.

*Ethyl 2-[acetyl(2-chlorobenzyl)amino]-7-chloro-6-hydroxy-1-benzothiophene-3-carboxylate **4e.***

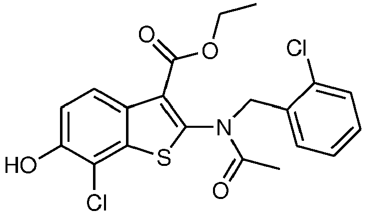

Yield: 15%. Mp. 174-178°C. Mass (EI), *m*/*z* (*Iᵣₑₗₐₜ*.(%)): 438.3247 [M]⁺ (55). C₂₀H₁₇Cl₂NO₄S. ¹H NMR (300 MHz, DMSO-d₆) δ 1.31 (t, *J* = 7.2Hz, 3H, CH₃CH₂), 2.00 (s, 3H, CH₃CO), 4.31 (d, *J* = 7.2 Hz, 2H, CH₃CH₂), 4.68, 5.25 (br. s, 2H, NCH₂), 7.17 (d, *J* = 8.9, 2.3 Hz, 1H, H5), 7.25 - 7.48 (m, 4H), 8.10 (d, *J* = 8.9 Hz, 1H, H4), 10.22 (br. s, 1H, OH).

### Example 10.

*Ethyl 2-[acetyl(2-chlorobenzyl)amino]-6-hydroxy-1-benzothiophene-3-carboxylate **5e**.*

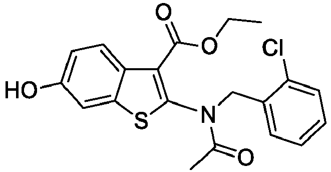

Yield: 24%. Mp. 176-180°C. Mass (EI), *m*/*z* (*Iᵣₑₗₐₜ.*(%)): 403.88 [M]⁺ (50). C₂₀H₁₈ClNO₄S. ¹H NMR (300 MHz, DMSO-d₆) δ 1.30 (t, *J* = 7.2Hz, 3H, CH₃CH₂), 1.98 (s, 3H, CH₃CO), 4.27 (q, *J* = 7.2 Hz, 2H, CH₃CH₂), 4.98 (br. s, 2H, NCH₂), 6.98 (dd, *J* = 8.9, 2.3 Hz, 1H, H5), 7.22 (d, *J* = 2.3 Hz, 1H, H7), 7.26 - 7.42 (m, 4H), 8.11 (d, *J* = 8.9 Hz, 1H, H4), 9.70 (s, 1H, OH).

### Example 11.

*Ethyl 2-[acetyl(4-chlorobenzyl)amino]-7-chloro-6-hydroxy-1-benzothiophene-3-carboxylate **4f.***

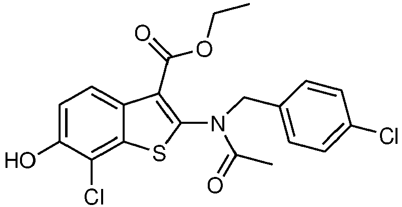

Yield: 11%. Mp. 174-176°C. Mass (EI), *m*/*z* (*Iᵣₑₗₐₜ*.(%)): 438.3247 [M]⁺ (43). C₂₀H₁₇Cl₂NO₄S. ¹H NMR (300 MHz, DMSO-d₆) δ 1.32 (t, *J* = 7.1 Hz, 3H, CH₃CH₂), 1.98 (s, 3H, CH₃CO), 4.19 (br. s, 2H, CH₂O), 4.70 (s, 1H, NCH₂), 4.90 (s, 1H, NCH₂), 7.11 - 7.25 (m, 4H), 7.40 (d, *J* = 8.0 Hz, 1H), 8.10 (d, *J* = 8.9 Hz, 1H, H4), 10.22 (s, 1H, OH).

### Example 12.

*Ethyl 2-[acetyl(4-chlorobenzyl)amino]-6-hydroxy-1-benzothiophene-3-carboxylate **5f.***

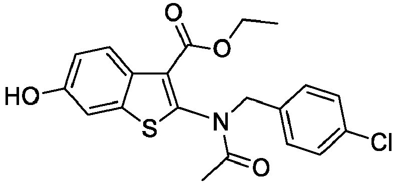

Yield: 45%. Mp. 172-176°C. Mass (EI), *m*/*z* (*Iᵣₑₗₐₜ*.(%)): 403.88 [M]⁺ (40). C₂₀H₁₈ClNO₄S. ¹H NMR (300 MHz, DMSO-d₆) δ 1.26 (t, *J* = 7.1 Hz, 3H, CH₃CH₂), 1.94 (s, 3H, CH₃CO), 4.19 (br. s, 2H, CH₂O), 4.60 (s, 1H, NCH₂), 5.02 (s, 1H, NCH₂), 6.98 (dd, *J* = 8.9, 2.4 Hz, 1H), 7.20 - 7.29 (m, 3H), 7.35 (d, *J* = 8.0 Hz, 2H), 8.10 (d, *J* = 8.9 Hz, 1H, H4), 9.84 (s, 1H, OH).

### Example 13.

*Ethyl 2-[acetyl(4-methylbenzyl)amino]-7-chloro-6-hydroxy-1-benzothiophene-3-carboxylate **4g**.*

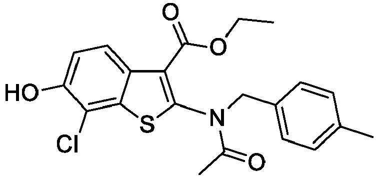

Yield: 22%. Mp. 170-172°C. Mass (EI), *mlz* (*Iᵣₑₗₐₜ*.(%)): 417.9065 [M]⁺ (40). C₂₁H₂₂ClNO₄S. ¹H NMR (300 MHz, DMSO-d₆) δ 1.25 (t, *J* = 7.1 Hz, 3H, CH₃CH₂), 1.95 (s, 3H, CH₃CO), 2.26 (s, 3H, CH₃Ph), 4.17 (br. s, 2H, CH₃CH₂), 4.61 (s, 1H, NCH₂), 4.97 (s, 1H, NCH₂), 7.09 (m, 4H), 7.19 (d, *J* = 8.9 Hz, 1H, H5), 8.07 (d, *J* = 8.9 Hz, 1H, H4), 10.67 (s, 1H, OH).

### Example 14.

*Ethyl 2-[acetyl(4-methylbenzyl)amino]-6-hydroxy-1-benzothiophene-3-carboxylate **5g**.*

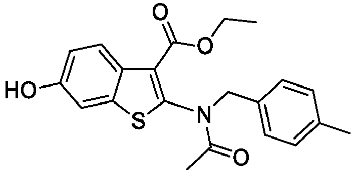

Yield: 20%. Mp. 145-147°C. Mass (EI), *m*/*z* (*Iᵣₑₗₐₜ*.(%)): 383.4618 [M]⁺ (38). C₂₁H₂₁NO₄S. ¹H NMR (300 MHz, DMSO-d₆) δ 1.25 (t, *J* = 7.1 Hz, 3H, CH₃CH₂), 1.95 (s, 3H, CH₃CO), 2.26 (s, 3H, CH₃Ph), 4.17 (br. s, 2H, CH₃CH₂), 4.50 (s, 1H, NCH₂), 5.05 (s, 1H, NCH₂), 6.98 (d, *J* = 8.9 Hz, 1H, H7), 7.11-7.30 (m, 5H), 8.11 (d, *J* = 8.9 Hz, 1H, H4), 9.97 (s, 1H, OH).

### Example 15.

*Methyl 2-[acetyl(2-chlorobenzyl)amino]-7-chloro-6-hydroxy-1-benzothiophene-3-carboxylate **4h.***

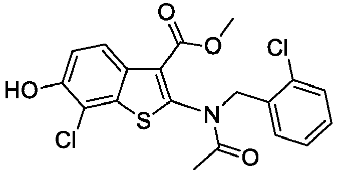

Yield: 45%. Mp. 229-233°C. Mass (EI), *m*/*z* (*Iᵣₑₗₐₜ*.(%)): 424.2981 [M]⁺ (60). C₁₉H₁₅Cl₂NO₄S. ¹H NMR (200 MHz, DMSO-d₆) δ 1.97 (s, 3H, CH₃CO), 3.73 (s, 3H, OCH₃), 4.50 (d, *J* = 15.4 Hz, 1H, NCH₂Ph), 5.42 (d, *J* = 15.4 Hz, 1H, NCH₂Ph), 7.18 (d, *J* = 8.9 Hz, 1H, H5), 7.25 -7.45 (m, 4H), 8.18 (d, *J* = 8.9 Hz, 1H, H4), 10.59 (s, 1H, OH).

### Example 16.

*Methyl 2-[acetyl(2-chlorobenzyl)amino]-6-hydroxy-1-benzothiophene-3-carboxylate **5h**.*

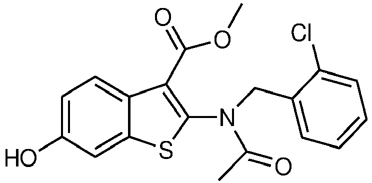

Yield: 2%. Mp. 220-224°C. Mass (EI), *m*/*z* (*Iᵣₑₗₐₜ*. (%)): 389.8534 [M]⁺ (68). C₁₉H₁₆ClNO₄S. ¹H NMR (200 MHz, DMSO-d₆) δ 1.97 (s, 3H, CH₃CO), 3.73 (s, 3H, OCH₃), 4.42 (d, *J* = 15.4 Hz, 1H, NCH₂Ph), 4.96 (d, *J* = 15.4 Hz, 1H, NCH₂Ph), 7.18 (d, *J* = 8.9 Hz, 1H, H5), 7.25 -7.45 (m, 4H), 7.36 (d, *J* = 3.3 Hz, 1H, H7), 8.18 (d, *J* = 8.9 Hz, 1H, H4), 9.65 (s, 1H, OH).

### Example 17.

*Methyl 2-[acetyl(4-methylbenzyl)amino]-7-chloro-6-hydroxy-1-benzothiophene-3-carboxylate **4i.***

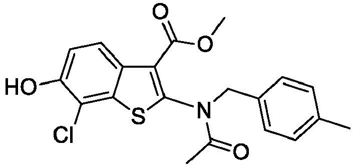

Yield: 22%. Mp. 195-197°C. Mass (EI), *m*/*z* (*Iᵣₑₗₐₜ*.(%)): 403.88 [M]⁺ (45). C₂₀H₁₈ClNO₄S. ¹H NMR (300 MHz, DMSO-d₆) δ 1.95 (s, 3H, CH₃CO), 2.26 (s, 3H, CH₃Ar), 3.72 (s, 3H, OCH₃), 4.64 (d, *J* = 13.4 Hz, 1H, NCH₂), 4.94 (d, *J* = 13.4 Hz, 1H, NCH₂), 7.06 - 7.11 (m, 4H), 7.18 (d, *J* = 8.9 Hz, 1H, H5), 8.04 (dd, *J* = 8.9, 1.7 Hz, 1H, H4), 10.72 (s, 1H, OH).

### Example 18.

*Methyl 2-[acetyl(4-methylbenzyl)amino]-6-hydroxy-1-benzothiophene-3-carboxylate **5i**.*

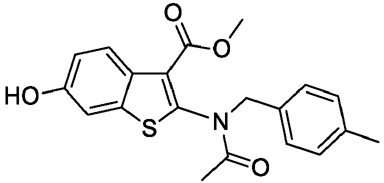

Yield: 20%. Mp. 160-164°C. Mass (EI), *m*/*z (Irelat.(%)):* 369.4352 [M]⁺ (50). C₂₀H₁₉NO₄S. ¹H NMR (300 MHz, DMSO-d₆) δ 1.93 (s, 3H, CH₃CO), 2.26 (s, 3H, CH₃Ar), 3.72 (s, 3H, OCH₃), 4.54 (d, *J* = 13.8 Hz, 1H, NCH₂), 4.99 (d, *J* = 13.8Hz, 1H, NCH₂), 6.97 (dd, *J* = 9.0, 2.4 Hz, 1H, H5), 7.06 - 7.14 (m, 4H), 7.26 (d, *J* = 2.3 Hz, 1H, H7), 8.06 (d, *J* = 8.9 Hz, 1H, H4), 9.88 (s, 1H, OH).

### Example 19.

*Methyl 2-[acetyl(2-trifluoromethylbenzyl)amino]-7-chloro-6-hydroxy-1-benzothiophene-3-carboxylate **4j.***

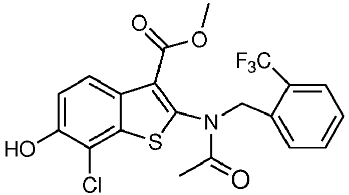

Yield: 41%. Mp. 182-186°C. Mass (EI), *m*/*z* (*Iᵣₑₗₐₜ*.(%)): 457.8513 [M]⁺ (55). C₂₀H₁₅ClF₃NO₄S. ¹H NMR (300 MHz, DMSO-d₆) δ 1.95 (s, 3H, CH₃CO), 3.70 (s, 3H, OCH₃), 4.90 (d, 2H, NCH₂), 7.20-7.67 (m, 5H), 8.05 (d, *J* = 8.9 Hz, 1H, H4), 10.70 (s, 1H, OH).

### Example 20.

*Methyl 2-[acetyl(2-trifluoromethylbenzyl)amino]-6-hydroxy-1-benzothiophene-3-carboxylate **5j**.*

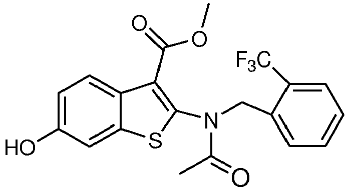

Yield: 20%. Mp. 165-169°C. Mass (EI), *mlz* (*Iᵣₑₗₐₜ*.(%)): 423.4066 [M]⁺ (60). C₂₀H₁₆F₃NO₄S. ¹H NMR (300 MHz, DMSO-d₆) δ 1.93 (s, 3H, CH₃CO), 3.72 (s, 3H, OCH₃), 4.72 (br. s, 1H, NCH₂), 5.02 (br. s, 1H, NCH₂), 6.98 (dd, *J* = 8.9, 2.3 Hz, 1H, H5), 7.28 (d, *J* = 2.3 Hz, 1H, H7), 7.44 (d, *J* = 8.0 Hz, 2H, H1', H4'), 7.67 (d, *J* = 8.0Hz, 2H, H2', H3'), 8.05 (d, *J* = 8.9 Hz, 1H, H4), 9.68 (s, 1H, OH).

### Example 21.

*Methyl 2-[acetyl(4-trifluoromethylbenzyl)amino]-7-chloro-6-hydroxy-1-benzothiophene-3-carboxylate **4k**.*

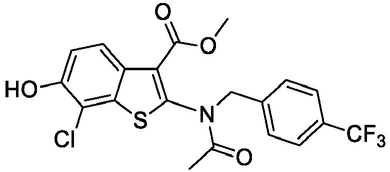

Yield: 35%. Mp. 176-180°C. Mass (EI), *m*/*z* (*Iᵣₑₗₐₜ*.(%)): 457.8513 [M]⁺ (55). C₂₀H₁₅ClF₃NO₄S. ¹H NMR (300 MHz, DMSO-d₆) δ 1.98 (s, 3H, CH₃CO), 3.70 (s, 3H, OCH₃), 4.93 (m, 2H, NCH₂), 7.20 (d, *J* = 8.9 Hz, 1H), 7.44 (d, *J* = 7.9 Hz, 2H, H1', H5'), 7.67 (d, *J* = 8.0 Hz, 2H, H2', H4'), 8.05 (d, *J* = 8.9 Hz, 1H, H4), 10.74 (s, 1H, OH).

### Example 22.

*Methyl 2-[acetyl(4-trifluoromethylbenzyl)amino]-6-hydroxy-1-benzothiophene-3-carboxylate **5k.***

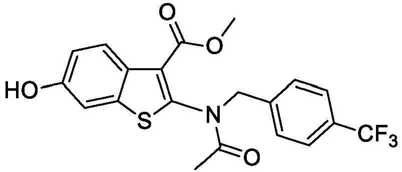

Yield: 17%. Mp. 171-175°C. Mass (EI), *m*/*z* (*Iᵣₑₗₐₜ*.(%)): 423.4066 [M]⁺ (58). C₂₀H₁₆F₃NO₄S. ¹H NMR (300 MHz, DMSO-d₆) δ 1.96 (s, 3H, CH₃CO), 3.71 (s, 3H, OCH₃), 4.78 (br. s, 1H, NCH₂), 5.05 (br. s, 1H, NCH₂), 6.98 (dd, *J* = 8.9, 2.3 Hz, 1H, H-5), 7.28 (d, *J* = 2.3 Hz, 1H, H7), 7.44 (d, *J* = 8.0 Hz, 2H, H1', H5'), 7.67 (d, *J* = 8.0Hz, 2H, H2', H4'), 8.07 (d, *J* = 8.9 Hz, 1H, H4), 9.88 (s, 1H, OH).

### Example 23.

*Methyl 2-[acetyl(2-fluorobenzyl)amino]-7-chloro-6-hydroxy-1-benzothiophene-3-carboxylate **4l.***

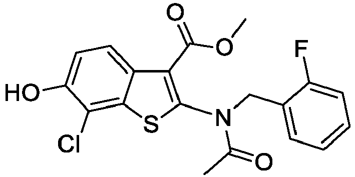

Yield: 25%. Mp. 229-232°C. Mass (EI), *m*/*z* (*Iᵣₑₗₐₜ*.(%)): 407.8438 [M]⁺ (45). C₁₉H₁₅ClFNO₄S. ¹H NMR (300 MHz, DMSO-cE) δ 1.97 (s, 3H, CH₃CO), 3.71 (s, 3H, OCH₃), 4.83 (d, *J* = 13.2 Hz, 1H, NCH₂), 4.98 (d, *J* = 13.2 Hz, 1H, NCH₂), 6.97- 7.43 (m, 5H), 8.05 (d, *J* = 8.9 Hz, 1H, H4), 10.73 (s, 1H, OH).

### Example 24.

*Methyl 2-[acetyl(2-fluorobenzyl)amino]-6-hydroxy-1-benzothiophene-3-carboxylate **5l.***

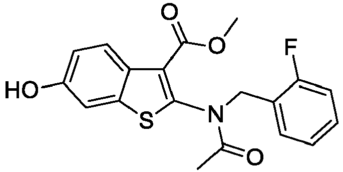

Yield: 41%. Mp. 165-167°C. Mass (EI), *mlz* (*Iᵣₑₗₐₜ*.(%)): 374.3991 [M]⁺ (50). C₁₉H₁₆FNO₄S. ¹H NMR (300 MHz, DMSO-d₆) δ 1.93 (s, 3H, CH₃CO), 3.71 (s, 3H, OCH₃), 4.78 (s, 1H, NCH₂), 4.99 (s, 1H, NCH₂), 6.98 (dd, *J* = 8.9, 2.4 Hz, 1H, H5), 7.04 - 7.17 (m, 2H), 7.23 - 7.39 (m, 3H), 8.07 (d, *J* = 8.9 Hz, 1H, H4), 9.90 (s, 1H, OH).

### Example 25.

*Methyl 2-[acetyl(3fluorobenzyl)amino]-7-chloro-6-hydroxy-1-benzothiophene-3-carboxylate **4m.***

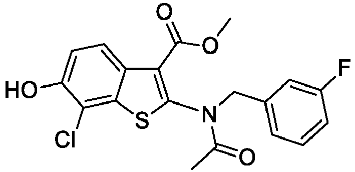

Yield: 35%. Mp. 152-155°C. Mass (EI), *mlz* (*Iᵣₑₗₐₜ*.(%)): 407.8438 [M]⁺ (20). C₁₉H₁₅ClFNO₄S. ¹H NMR (300 MHz, DMSO-cE) δ 1.98 (s, 3H, CH₃CO), 3.74 (s, 3H, OCH₃), 4.77 (s, 1H, NCH₂), 4.94 (s, 1H, NCH₂), 7.07 (m, 3H), 7.20 (d, *J* = 8.9 Hz, 1H, H5), 7.29 - 7.39 (m, 1H), 8.05 (d, *J* = 8.9 Hz, 1H, H4), 10.70 (s, 1H, OH).

### Example 26.

*Methyl 2-[acetyl(3-fluorobenzyl)amino]-6-hydroxy-1-benzothiophene-3-carboxylate **5m.***

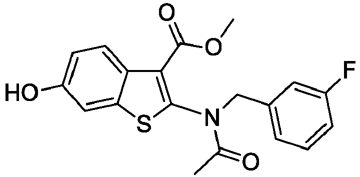

Yield: 30%. Mp. 145-150°C. Mass (EI), *mlz* (*Iᵣₑₗₐₜ*.(%)): 374.3991 [M]⁺ (18). C₁₉H₁₆FNO₄S. ¹H NMR (300 MHz, DMSO-d₆) δ 1.96 (s, 3H, CH₃CO), 3.74 (s, 3H, OCH₃), 4.68 (s, 1H, NCH₂), 4.99 (s, 1H, NCH₂), 6.98 (dd, *J* = 8.9, 2.5 Hz, 1H), 7.01 - 7.07 (m, 2H), 7.10 (dd, *J* = 8.9, 2.5 Hz, 1H, H5), 7.28 (d, *J* = 2.3 Hz, 1H, H7), 7.27 - 7.40 (m, 1H), 8.07 (d, *J* = 8.9 Hz, 1H, H4), 9.88 (s, 1H, OH).

### Example 27.

*Methyl 2-[acetyl(4-fluorobenzyl)amino]-7-chloro-6-hydroxy-1-benzothiophene-3-carboxylate **4n.***

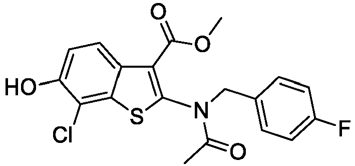

Yield: 27%. Mp. 233-235°C. Mass (EI), *m*/*z* (*Iᵣₑₗₐₜ*.(%)): 407.8438 [M]⁺ (19). C₁₉H₁₅ClFNO₄S. ¹H NMR (300 MHz, DMSO-cE) δ 1.96 (s, 3H, CH₃CO), 3.72 (s, 3H, OCH₃), 4.83 (br. s, 2H, NCH₂), 7.37 - 6.97 (m, 5H), 8.04 (d, *J* = 8.9 Hz, 1H, H4), 10.69 (s, 1H, OH).

### Example 28.

*Methyl 2-[acetyl(4-fluorobenzyl)amino]-6-hydroxy-1-benzothiophene-3-carboxylate **5n**.*

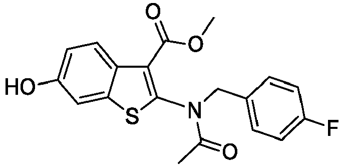

Yield: 29%. Mp. 198-202°C. Mass (EI), *m*/*z* (*Iᵣₑₗₐₜ*.(%)): 374.3991 [M]⁺ (22). C₁₉H₁₆FNO₄S. ¹H NMR (300 MHz, DMSO-d₆) δ 1.93 (s, 3H, CH₃CO), 3.72 (s, 3H, OCH₃), 4.65 (d, *J* = 13.1Hz, 1H, NCH₂), 4.95 (d, *J* = 13.1 Hz, 1H, NCH₂), 6.97 (dd, *J* = 9.0, 2.3 Hz, 1H, H5), 7.10 (t, *J* = 8.7 Hz, 2H, H3', H5'), 7.19 - 7.29 (m, 3H), 8.06 (d, *J* = 8.9 Hz, 1H, H4), 9.91 (s, 1H, OH).

### Example 29.

*Methyl 2-[acetyl(2,4-difluorobenzyl)amino]-7-chloro-6-hydroxy-1-benzothiophene-3-carboxylate **4o**.*

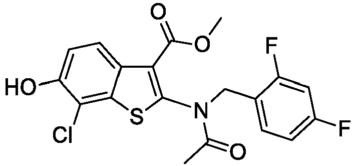

Yield: 41%. Mp. 205-210°C. Mass (EI), *m*/*z* (*Iᵣₑₗₐₜ*.(%)): 425.8343 [M]⁺ (20). C₁₉H₁₄ClF₂NO₄S. ¹H NMR (200 MHz, DMSO-d₆) δ 1.94 (s, 3H, CH₃CO), 3.71 (s, 3H, OCH₃), 4.88 (br. s, 2H, NCH₂Ph), 7.02 (tdd, *J* = 8.5, 2.8, 0.9 Hz, 1H), 7.10 - 7.24 (m, 1H), 7.19 (d, *J* = 8.9 Hz, 1H, H5), 7.33 (td, *J* = 8.6, 6.7 Hz, 1H, H6'), 8.05 (d, *J* = 8.9 Hz, 1H, H4), 10.78 (s, 1H, OH).

### Example 30.

*Methyl 2-[acetyl(2,4-difluorobenzyl)amino]-6-hydroxy-1-benzothiophene-3-carboxylate **5o.***

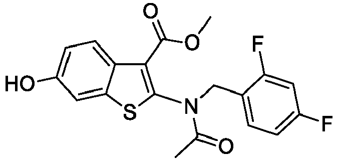

Yield: 15%. Mp. 178-180°C. Mass (EI), *m*/*z* (*Iᵣₑₗₐₜ*.(%)): 391.3895 [M]⁺ (24). C₁₉H₁₅F₂NO₄S. ¹H NMR (200 MHz, DMSO-d₆) δ 1.92 (s, 3H, CH₃CO), 3.72 (s, 3H, OCH₃), 4.78 (d, *J* = 13.0 Hz, 1H, NCH₂Ph), 4.93 (d, *J* = 13.0 Hz, 1H, NCH₂Ph), 6.85-7.44 (m, 5H), 8.06 (d, *J* = 8.9 Hz, 1H, H4), 9.92 (s, 1H, OH).

### Example 31.

*Methyl 2-[acetyl(1-naphthylmethyl)amino]-7-chloro-6-hydroxy-1-benzothiophene-3-carboxylate **4p**.*

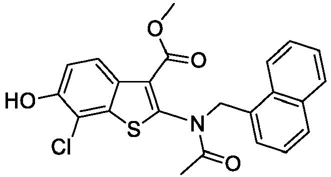

Yield: 28%. Mp. 210-212°C. Mass (EI), *m*/*z* (*Iᵣₑₗₐₜ*.(%)): 439.9121 [M]⁺ (35). C₂₃H₁₈ClNO₄S. ¹H NMR (300 MHz, DMSO-d₆) δ 1.99 (s, 3H, CH₃CO), 3.39 (s, 3H, OCH₃), 5.34 (br. s, 2H, NCH₂), 7.14 (d, *J* = 8.8 Hz, 2H), 7.30 (t, *J* = 7.6 Hz, 1H), 7.54 (td, *J* = 6.8, 6.4, 3.5 Hz, 2H), 7.84 (d, *J* = 8.2 Hz, 1H), 7.91 - 7.99 (m, 2H), 8.13 (d, *J* = 8.8Hz, 1H, H4), 10.68 (s, 1H, OH).

### Example 32.

*Methyl 2-[acetyl(1-naphthylmethyl)amino]-6-hydroxy-1-benzothiophene-3-carboxylate **5p.***

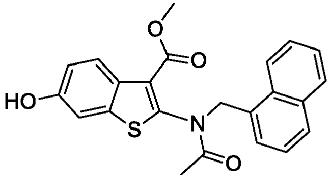

Yield: 22%. Mp. 198-202°C. Mass (EI), *m*/*z* (*Iᵣₑₗₐₜ*.(%)): 405.4673 [M]⁺ (42). C₂₃H₁₉NO₄S. ¹H NMR (300 MHz, DMSO-d₆) δ 1.96 (s, 3H, CH₃CO), 3.45 (s, 3H, OCH₃), 5.20 (d, *J* = 14.0 Hz, 1H, NCH₂), 5.43 (d, *J* = 14.0 Hz, 1H, NCH₂), 6.93 (dd, *J* = 8.9, 2.3 Hz, 1H, H5), 7.12 (d, *J* = 7.0 Hz, 1H), 7.18 (d, *J* = 2.3 Hz, 1H, H7), 7.29 (t, *J* = 7.6 Hz, 1H), 7.48 - 7.60 (m, 2H), 7.83 (d, *J* = 8.2 Hz, 1H), 7.89 - 7.95 (m, 1H), 7.98 (d, *J* = 8.9 Hz, 1H), 8.13 (d, *J* = 8.8Hz, 1H, H4), 9.86 (s, 1H, OH).

### Example 33.

*Methyl 2-[acetyl(2-naphthylmethyl)amino]-7-chloro-6-hydroxy-1-benzothiophene-3-carboxylate **4q.***

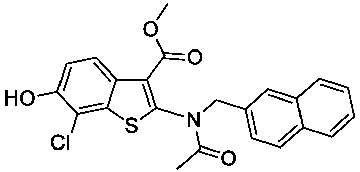

Yield: 24%. Mp. 185-190°C. Mass (EI), *m*/*z* (*Iᵣₑₗₐₜ*.(%)): 439.9121 [M]⁺ (40). C₂₃H₁₈ClNO₄S.

¹H NMR (300 MHz, DMSO-d₆) δ 2.00 (s, 3H, CH₃CO), 3.64 (s, 3H, OCH₃), 4.89 (d, *J* = 12.6 Hz, 1H, NCH₂), 5.15 (d, *J* = 13.6 Hz, 1H, NCH₂), 7.17 (d, *J* = 8.9 Hz, 1H, H5), 7.38 (d, *J* = 8.5 Hz, 1H), 7.44 - 7.53 (m, 2H), 7.69 (s, 1H, H2'), 7.85 (dt, *J* = 13.5, 5.3 Hz, 3H), 8.01 (d, *J* = 8.9 Hz, 1H, H4), 10.72 (s, 1H, OH).

### Example 34.

*Methyl 2-[acetyl(2-naphthylmethyl)amino]-6-hydroxy-1-benzothiophene-3-carboxylate **5q**.*

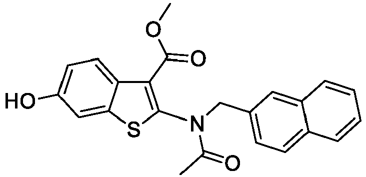

Yield: 23%. Mp. 200-202°C. Mass (EI), *m*/*z* (*Iᵣₑₗₐₜ*.(%)): 405.4673 [M]⁺ (38). C₂₃H₁₉NO₄S.

¹H NMR (300 MHz, DMSO-d₆) δ 1.98 (s, 3H, CH₃CO), 3.68 (s, 3H, OCH₃), 4.76 (d, *J* = 12.2 Hz, 1H, NCH₂), 5.23 (d, *J* = 12.2 Hz, 1H, NCH₂), 6.96 (dd, *J* = 9.0, 2.4 Hz, 1H, H5), 7.23 (d, *J* = 2.3 Hz, 1H), 7.38 (dd, *J* = 8.5, 1.8 Hz, 1H), 7.44 - 7.52 (m, 2H), 7.68 (s, 1H, H2'), 7.78 - 7.91 (m, 3H), 8.05 (d, *J* = 8.9 Hz, 1H, H4), 9.86 (s, 1H, OH).

### Example 35.

*Methyl 2-[acetyl(2-phenylethyl)amino]-7-chloro-6-hydroxy-1-benzothiophene-3-carboxylate **4r**.*

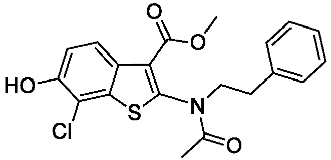

Yield: 9%. Mp. 150-155°C. Mass (EI), *m*/*z* (*Iᵣₑₗₐₜ.*(%)): 403.88 [M]⁺ (53). C₂₀H₁₈ClNO₄S.

¹H NMR (200 MHz, DMSO-d₆) δ 1.90 (s, 3H, CH₃CO), 2.64 - 3.00 (m, 3H, PhCH₂, NCH), 3.58 - 3.76 (m, 1H, NCH), 3.84 (s, 3H, OCH₃), 3.93 - 4.12 (m, 1H, NCH), 7.07 - 7.40 (m, 6H), 8.14 (d, *J* = 8.9 Hz, 1H, H4), 10.79 (s, 1H, OH).

### Example 36.

*Methyl 2-[acetyl(2-phenylethyl)amino]-6-hydroxy-1-benzothiophene-3-carboxylate **5r.***

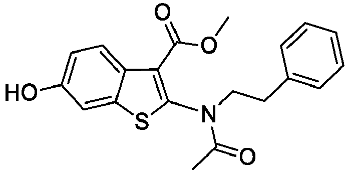

Yield: 11%. Mp. 190-194°C. Mass (EI), *m*/*z* (*Iᵣₑₗₐₜ*.(%)): 369.4352 [M]⁺ (59). C₂₀H₁₉NO₄S.¹H NMR (200 MHz, DMSO-d₆) δ 1.88 (s, 3H, CH₃CO), 2.84 (s, 2H, PhCH₂), 3.62 (s, 2H, NCH₂), 3.83 (s, 3H, OCH₃), 7.02 (dd, *J* = 8.9, 2.4 Hz, 1H, H5), 7.12 - 7.36 (m, 6H), 8.15 (d, *J* = 8.9 Hz, 1H, H4), 10.02 (s, 1H, OH).

### Example 37.

*Methyl 2-[acetyl(1-phenylethyl)amino]-7-chloro-6-hydroxy-1-benzothiophene-3-carboxylate **4s.***

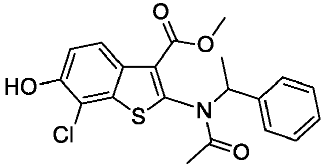

Yield: 8%. Mp. 195-200°C. Mass (EI), *m*/*z* (*Iᵣₑₗₐₜ*.(%)): 403.88 [M]⁺ (66). C₂₀H₁₈ClNO₄S. ¹H NMR (200 MHz, DMSO-d₆) δ 1.28 (d, *J* = 7.2 Hz, 2H, CH₃CH), 1.58 (d, *J* = 7.1 Hz, 1H, CH₃CH), 1.91 (s, 3H, CH₃CO), 3.48 (s, 1H, OCH₃), 3.90 (s, 2H, OCH₃), 6.07 (br. q, *J* = 8.1 Hz, 1H, CH₃CH), 7.45 - 7.05 (m, 6H), 7.82 (d, *J* = 8.9 Hz, 0.4H, H4), 8.08 (d, *J* = 8.9 Hz, 0.6H, H4), 10.75 (s, 1H, OH). (Mixture of stereoisomers).

### Example 38.

*Methyl 2-[acetyl(1-phenylethyl)amino]-6-hydroxy-1-benzothiophene-3-carboxylate **5s.***

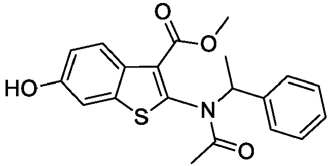

Yield: 14%. Mp. 152-157°C. Mass (EI), *m*/*z* (*Iᵣₑₗₐₜ*.(%)): 369.4352 [M]⁺ (64). C₂₀H₁₉NO₄S. ¹H NMR (200 MHz, DMSO-d₆) δ 1.27 (d, *J* = 7.3 Hz, 2H, CH₃CH), 1.56 (d, *J* = 7.1 Hz, 1H, CH₃CH), 1.90 (s, 3H, CH₃CO), 3.47 (s, 1H, OCH₃), 3.89 (s, 2H, OCH₃), 6.04 (br. q, *J* = 7.2Hz, 1H, CH₃CH), 7.08 - 7.43 (m, 6H), 7.83 (d, *J* = 9.0 Hz, 0.4H, H4), 8.09 (d, *J* = 8.8 Hz, 0.6H, H4), 9.90 (s, 1H, OH). (Mixture of stereoisomers).

### Example 39.

*Methyl 2-[acetyl(2-methylbenzyl)amino]-7-chloro-6-hydroxy-1-benzothiophene-3-carboxylate **4t**.*

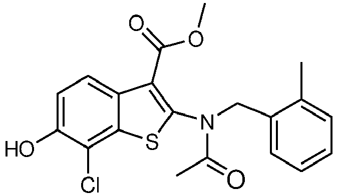

Yield: 28%. Mp. 182-184°C. Mass (EI), *m*/*z* (*Iᵣₑₗₐₜ*.(%)): 403.88 [M]⁺ (38). C₂₀H₁₈ClNO₄S. ¹H NMR (300 MHz, DMSO-d₆) δ 1.95 (s, 3H, CH₃CO), 2.25 (s, 3H, CH₃Ar), 3.72 (s, 3H, OCH₃), 4.64 (d, *J* = 13.4 Hz, 1H, NCH₂), 4.94 (d, *J* = 13.4 Hz, 1H, NCH₂), 7.12 - 7.20 (m, 4H), 7.22 (d, *J* = 8.9 Hz, 1H, H5), 8.12 (dd, *J* = 8.9, 1.7 Hz, 1H, H4), 10.72 (s, 1H, OH).

### Example 40.

*Methyl 2-[acetyl(2-methylbenzyl)amino]-6-hydroxy-1-benzothiophene-3-carboxylate **5t.***

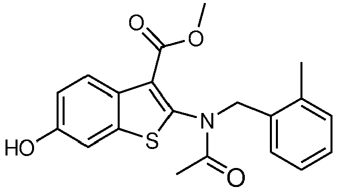

Yield: 26%. Mp. 164-168°C. Mass (EI), *m*/*z* (*Iᵣₑₗₐₜ*.(%)): 369.4352 [M]⁺ (40). C₂₀H₁₉NO₄S. ¹H NMR (300 MHz, DMSO-d₆) δ 1.93 (s, 3H, CH₃CO), 2.26 (s, 3H, CH₃Ar), 3.72 (s, 3H, OCH₃), 4.54 (d, *J* = 13.8 Hz, 1H, NCH₂), 5.02 (d, *J* = 13.8Hz, 1H, NCH₂), 6.99 (dd, *J* = 9.0, 2.4 Hz, 1H, H5), 7.06 - 7.14 (m, 4H), 7.26 (d, *J* = 2.3 Hz, 1H, H7), 8.06 (d, *J* = 8.9 Hz, 1H, H4), 9.88 (s, 1H, OH).

### Example 41.

*Methyl 2-[acetyl(4-isopropylbenzyl)amino]-7-chloro-6-hydroxy-1-benzothiophene-3-carboxylate **4u.***

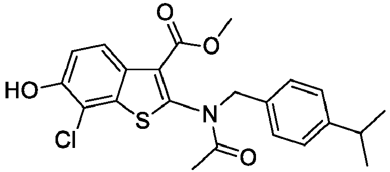

Yield: 8%. Mp. 183-186°C. Mass (EI), *m*/*z* (*Iᵣₑₗₐₜ*.(%)): 431.9331 [M]⁺ (50). C₂₂H₂₂ClNO₄S. ¹H NMR (200 MHz, DMSO-d₆) δ 1.20 (d, *J* = 6.7 Hz, 6H, (CH₃)₂), 1.98 (s, 3H, CH₃CO), 2.82 (hept, *J* = 6.7 Hz, 1H, CH(CH₃)₂), 3.72 (s, 3H, OCH3), 4.60 (br. s, 1H, NCH₂), 4.80 (br. s, 1H, NCH₂), 7.00 - 7.20 (m, 5H), 8.04 (d, *J* = 8.7Hz, 1H, H4), 10.2 (s, 1H, OH).

### Example 42.

*Methyl 2-[acetyl(4-isopropylbenzyl)amino]-6-hydroxy-1-benzothiophene-3-carboxylate **5u.***

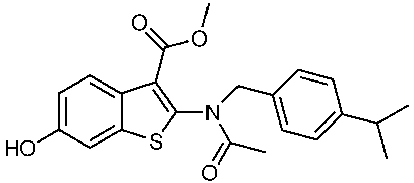

Yield: 30%. Mp. 141-145°C. Mass (EI), *m*/*z* (*Iᵣₑₗₐₜ*.(%)): 397.4884 [M]⁺ (59). C₂₂H₂₃NO₄S. ¹H NMR (200 MHz, DMSO-d₆) δ 1.20 (d, *J* = 6.7 Hz, 6H, (CH₃)₂), 1.98 (s, 3H, CH₃CO), 2.82 (hept, *J* = 6.7 Hz, 1H, CH(CH₃)₂), 3.72 (s, 3H, OCH3), 4.65 (br. s, 1H, NCH₂), 4.82 (br. s, 1H, NCH₂), 6.98 (dd, *J* = 8.7, 2.4 Hz, 1H, H5), 7.00 - 7.20 (m, 4H), 7.25 (s, 1H, H7), 8.11 (d, *J* = 8.7Hz, 1H, H4), 9.88 (s, 1H, OH).

### Example 43.

*Methyl 2-[acetyl(2-cyanobenzyl)amino]-7-chloro-6-hydroxy-1-benzothiophene-3-carboxylate **4v.***

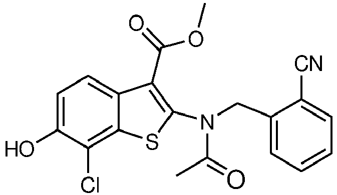

Yield: 28%. Mp. 204-208°C. Mass (EI), *mlz* (*Iᵣₑₗₐₜ*.(%)): 414.8629 [M]⁺ (35). C₂₀H₁₅ClN₂O₄S. ¹H NMR (300 MHz, DMSO-d₆) δ 1.97 (s, 3H, CH₃CO), 3.71 (s, 3H, OCH₃), 4.80 (d, *J* = 13.2 Hz, 1H, NCH₂), 4.95 (d, *J* = 13.2 Hz, 1H, NCH₂), 7.01- 7.47 (m, 5H), 8.06 (d, *J* = 8.9 Hz, 1H, H4), 10.81 (s, 1H, OH).

### Example 44.

*Methyl 2-[acetyl(2-cyanobenzyl)amino]-6-hydroxy-1-benzothiophene-3-carboxylate **5v**.*

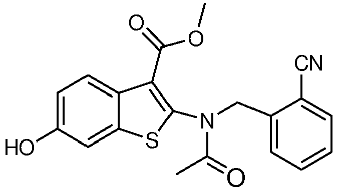

Yield: 38%. Mp. 168-172°C. Mass (EI), *m*/*z* (*Iᵣₑₗₐₜ*.(%)): 380.4181 [M]⁺ (54). C₂₀H₁₆FN₂O₄S. ¹H NMR (300 MHz, DMSO-d₆) δ 1.93 (s, 3H, CH₃CO), 3.71 (s, 3H, OCH₃), 4.88 (s, 1H, NCH₂), 5.10 (s, 1H, NCH₂), 7.05 (dd, *J* = 8.9, 2.4 Hz, 1H, H5), 7.08 - 7.20 (m, 2H), 7.28 - 7.44 (m, 3H), 8.10 (d, *J* = 8.9 Hz, 1H, H4), 9.88 (s, 1H, OH).

### Example 45.

*Methyl 2-[acetyl(2-fluorophenylethyl)amino]-7-chloro-6-hydroxy-1-benzothiophene-3-carboxylate **4w.***

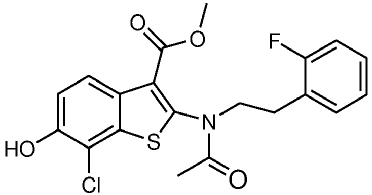

Yield: 15%. Mp. 172-174°C. Mass (EI), *m*/*z* (*Iᵣₑₗₐₜ*.(%)): 421.8704 [M]⁺ (43). C₂₀H₁₇ClFNO₄S.

¹H NMR (200 MHz, DMSO-d₆) δ 1.90 (s, 3H, CH₃CO), 2.66 - 3.02 (m, 2H, PhCH₂, NCH₂), 3.59 - 3.78 (m, 1H, NCH₂), 3.84 (s, 3H, OCH₃), 3.94 - 4.13 (m, 1H, NCH₂), 7.09 - 7.43 (m, 5H), 8.16 (d, *J* = 8.9 Hz, 1H, H4), 10.79 (s, 1H, OH).

### Example 46.

*Methyl 2-[acetyl(2-fluorophenylethyl)amino]-6-hydroxy-1-benzothiophene-3-carboxylate **5w.***

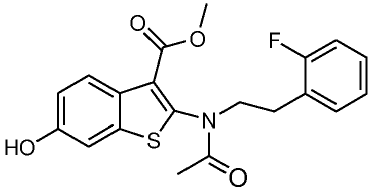

Yield: 12%. Mp. 183-185°C. Mass (EI), *m*/*z* (*Iᵣₑₗₐₜ*.(%)): 387.4257 [M]⁺ (49). C₂₀H₁₈FNO₄S. ¹H NMR (200 MHz, DMSO-d₆) δ 1.88 (s, 3H, CH₃CO), 2.86 (s, 2H, PhCH₂), 3.64 (s, 2H, NCH₂), 3.83 (s, 3H, OCH₃), 7.10 (dd, *J* = 8.9, 2.4 Hz, 1H, H5), 7.14 - 7.38 (m, 5H), 8.16 (d, *J* = 8.9 Hz, 1H, H4), 10.00 (s, 1H, OH).

### General synthesis method 2.

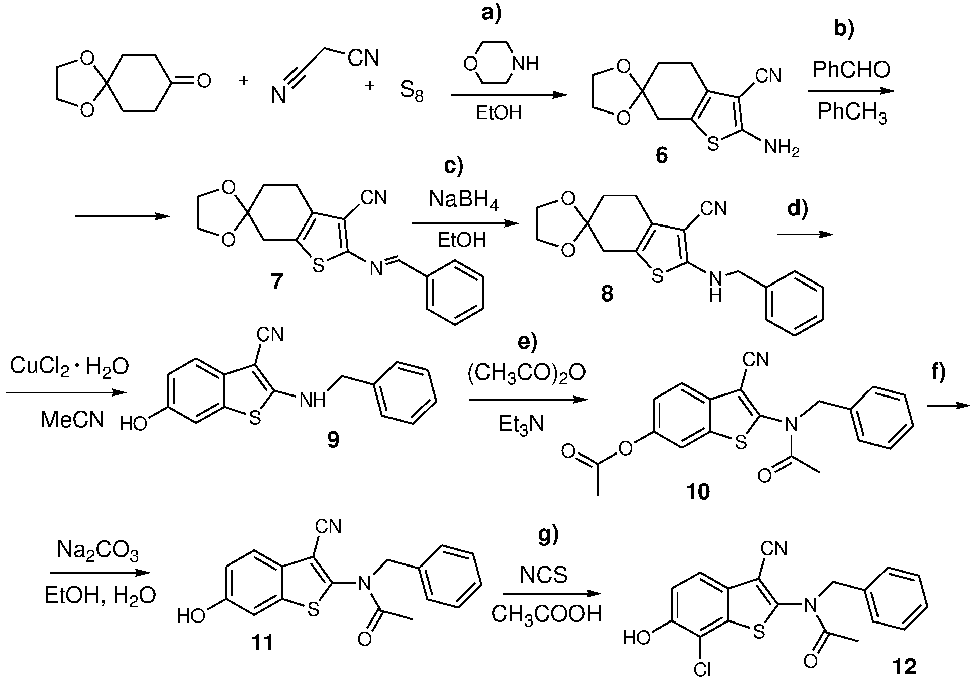

### a) Preparation of 2-amino-4,1-dihydro-5H-spiro[1-benzothiophene-6,2'-[1,3]dioxolane]-3-carbonitrile 6.

A mixture of 1,4-dioxospiro[4.5]decan-8-one (6.24 g, 40 mmol), malononitrile (2.64 g, 40 mmol), sulfur (1.28 g, 40 mmol) and morpholine (3.40 ml, 40 mmol) in 40 ml of ethanol is stirred for 2 hours at a temperature of 70°C and cooled. The resulting precipitate is filtered off, washed with ethanol, and crystallized from a mixture of toluene and acetone. Yield: 71%. Mp. 193-195°C. Mass (EI), *m*/*z* (*Iᵣₑₗₐₜ.*(%)): 236.2913 [M]⁺ (73). C₁₁H₁₂N₂O₂S.

### b) Preparation of 2-{[(1E)-phenylmethylene]amino]-4,7-dihydro-5H-spiro[1-benzothiophene-6,2'-[1,3]dioxolane]-3-carbonitrile 7.

Compound **6** (7.08 g, 30.0 mmol), benzaldehyde (3.45 g, 33.0 mmol) and 0.1 g p-TsOH are refluxed with stirring in 70 ml of toluene for 1 h with a nozzle for separating water. Toluene is evaporated to a volume of ~20 ml, the precipitate is filtered off and washed with toluene and hexane. Yield: 90%. Mp. 137-139°C. Mass (EI), *m*/*z* (*Iᵣₑₗₐₜ*.(%)): 324.3979 [M]⁺ (36). C₁₈H₁₆N₂O₂S.

### c) Preparation of 2-(benzylamino)-4,7-dihydro-5H-spiro[1- benzothiophene-6,2'-[1,3]dioxolane]-3-carbonitrile 8.

A suspension of compound **7** (3.24 g, 10 mmol) and sodium borohydride (0.380 g, 10 mmol) is stirred in 50 ml of ethanol at room temperature for 15 min and then for 30 min at 500C. Ethanol is evaporated to a volume of 15 ml, 20 ml of water is added to the residue, the precipitate is filtered off and washed with 30% ethanol and water. Yield: 88%. Mp. 134-136°C. Mass (EI), *m*/*z* (*Iᵣₑₗₐₜ.*(%)): 326.4138 [M]⁺ (46). C₁₈H₁₈N₂O₂S.

### d) Preparation of 2-(benzylamino)-6-hydroxy-1-benzothiophene-3-carbonitrile 9.

200 ml of acetonitrile is added to a mixture of compound **8** (9.78 g, 30.0 mmol) and CuCl₂·2H₂O (10.2 g, 60.0 mmol). The suspension is stirred at room temperature for 45 min, and 330 ml of 0.05 M hydrochloric acid and 330 ml of ethyl acetate are added to the reaction mixture. The aqueous layer is separated, the extract is washed with water (3×200 ml) to pH~6.5 and dried with sodium sulfate. Ethyl acetate is evaporated, 30 ml of a mixture of chloroform and acetone (20:1) is added to the residue, the precipitate is stirred, filtered, and washed with chloroform. Yield: 39%. Mp. 167-169°C. Mass (EI), *m*/*z* (*Iᵣₑₗₐₜ*.(%)): 280.3454 [M]⁺ (53). C₁₆H₁₂N₂OS.

### e) Preparation of 2-[acetyl(benzyl)amino]-3-cyano-1-benzothiophene-6-yl acetate 10.

Compound **9** (2.80 g, 10.0 mmol) in 15 ml of acetic anhydride and 2 ml of triethylamine is refluxed for 30 min with stirring. Acetic anhydride is decomposed with water. The reaction mixture is evaporated, the residue is dissolved in ethyl acetate and washed with water. The solution is dried with sodium sulfate. Ethyl acetate is evaporated off and the oil residue is used in the next step without further purification. Yield: 94%. oil. Mass (EI), *m*/*z* (*Iᵣₑₗₐₜ*.(%)): 364.4187 [M]⁺ (39). C₂₀H₁₆N₂O₃S.

### Example 47.

*N-benzyl- N-(3-cyano-6-hydroxy-1-benzothiophene-2-yl)acetamide **11.***

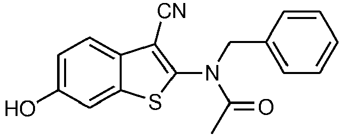

Method f) Compound **10** (3.64 g, 10.0 mmol) in 40 ml of ethanol is added to a solution of 6 g of sodium carbonate in 20 ml of water. The suspension is boiled with stirring for 30 minutes. Volatiles are removed under vacuum. Ethyl acetate and water are added to the residue, the mixture is stirred, the aqueous layer is separated, the extract is washed with water to pH ~ 6.5 and dried with sodium sulfate. Ethyl acetate is evaporated, the residue in the form of oil is used in the next step without further purification. Yield: 88%. oil. Mass (EI), *m*/*z* (*Iᵣₑₗₐₜ.*(%)): 322.3820 [M]⁺ (50). C₁₈H₁₄N₂O₂S. ¹H NMR (300 MHz, DMSO-d₆) δ 2.11 (br. s, 3H, CH₃CO), 4.98 (br. s, 2H, NCH₂), 7.04 (dd, *J* = 8.7, 2.2 Hz, 1H, H5), 7.16 - 7.46 (m, 6H), 7.61 (d, *J* = 8.8 Hz, 1H, H4), 10.07 (s, 1H, OH).

### Example 48.

g) *N-benzyl- N-(7-chloro-3-cyano-6-hydroxy-1-benzothiophene-2-yl)acetamide **12.***

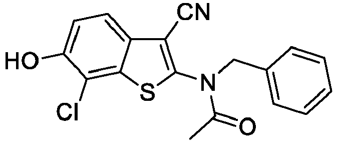

0.222 g (1.66 mmol) of chlorosuccinimide is added to a solution of compound **11** (0.5 g, 1.55 mmol) in 5 ml of acetic acid. The mixture is stirred at room temperature for 1 hour and water is poured out. The dark oil is extracted with ethyl acetate, the aqueous layer is separated, the extract is washed with water to pH 6.5 and dried over sodium sulfate. Ethyl acetate is evaporated. The residue is stirred in an ethanol-water-hexane mixture. The crystals are filtered and washed with aqueous ethanol. The product is purified by crystallization from toluene. Yield: 50%. Mp. 176-178°C. Mass (EI), *m*/*z* (*Iᵣₑₗₐₜ*.(%)): 356.8268 [M]⁺ (46). C₁₈H₁₃ClN₂O₂S. ¹H NMR (200 MHz, DMSO-d₆) δ 2.14 (s, 3H, CH₃CO), 5.03 (s, 2H, NCH₂Ph), 7.27 (m, 6H), 7.62 (d, *J* = 8.7 Hz, 1H, H4), 10.98 (s, 1H, OH).

### General synthesis method 3.

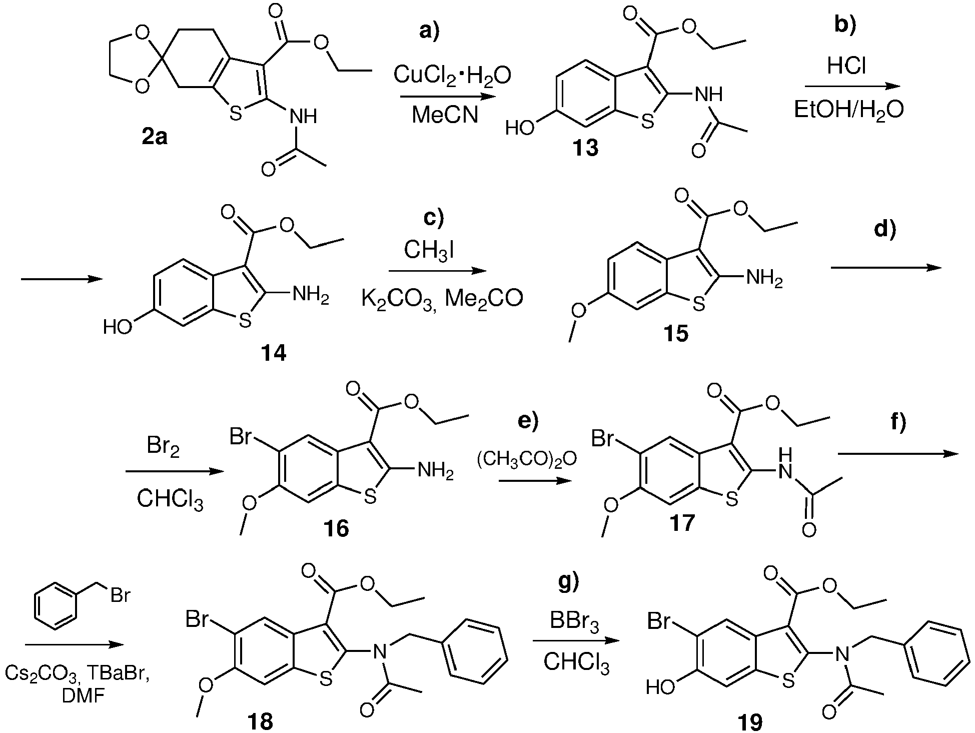

### a) Preparation of ethyl 2-(acetylamino)-6-hydroxy-1-benzothiophene-3-carboxylate 13.

CuCl₂·2H₂O (11.9 g, 70.0 mmol) is added to a solution of compound **2a** (9.6 g, 20.0 mmol) in 200 ml of acetonitrile. The suspension is stirred at room temperature for 2 hours, and 330 ml of 0.05 M hydrochloric acid and 330 ml of ethyl acetate are added to the reaction mixture. The aqueous layer is separated, the extract is washed with water (3×200 ml) to pH~6.5 and dried with sodium sulfate. Ethyl acetate is evaporated, 40 ml of diethyl ether is added to the residue, the precipitate is filtered off and washed with ethyl alcohol and diethyl ether and crystallized from ethyl alcohol. Yield: 78%. Mp. 210-213°C. Mass (EI), *m*/*z* (*I_{relat.}*(%)): 279.3127 [M]⁺ (65). C₁₃H₁₃NO₄S.

### b) Preparation of ethyl 2-amino-6-hydroxy-1-benzothiophene-3-carboxylate 14.

The reaction is carried out in a bomb previously purged with argon. Compound **13** (1 g, 4 mmol) is dissolved in 10 ml of 2 N HCl and 50 ml of ethyl alcohol, the solution is heated on a silicone bath at the temperature of 100°C for 20 hours. The reaction mixture is cooled and neutralized with ammonium acetate (2 g), the solution is evaporated. Water is added to the residue, cooled, and the reaction product gradually crystallizes. The precipitate is filtered off, washed with water, and crystallized from toluene. Yield: 88%. Mp. 170-175°C. Mass (EI), *m*/*z* (*I_{relat.}*(%)): 237.276 [M]⁺ (53). C₁₁H₁₁NO₃S.

### c) Preparation of ethyl 2-amino-6-methoxy-1-benzothiophene-3-carboxylate 15.

The reaction is carried out in a bomb. Compound **14** (1 g, 4 mmol) is dissolved in 20 ml of dry acetone, potassium carbonate (0.7 g, 4.8 mmol) and methyl iodide (0.32 ml, 4.8 mmol) are added. The solution is kept on a silicone bath at the temperature of 80°C for 20 hours. The reaction mixture is cooled, acetone is evaporated, water is added to the oily residue. Decantation is carried out. The precipitate crystallizes gradually, it is filtered off, washed with water, and purified by column chromatography using chloroform as the eluent. A by-product of the reaction is ethyl 6-methoxy-2-(methylamino)-1-benzothiophene-3-carboxylate, which is formed with a yield of 3%. The yield of product **15** is 77%. Mp. 95-97°C. Mass (EI), *m*/*z* (*Iᵣₑₗₐₜ*.(%)): 251.3026 [M]⁺ (75). C₁₂H₁₃NO₃S.

### d) Preparation of ethyl 2-amino-5-bromo-6-methoxy-1-benzothiophene-3-carboxylate 16.

A solution of bromine (0.1 ml, 2 mmol) in 3 ml of chloroform is added dropwise a solution of compound **15** (0.22 g, 0.8 mmol) in 5 ml of chloroform with stirring at room temperature. The reaction mixture is boiled for 1 hour and cooled, the resulting precipitate is filtered off, washed with chloroform, transferred to an aqueous solution of ammonium acetate (0.4 g), and stirred for 30 min. The precipitate is filtered off, washed with water, methyl alcohol, and hexane. Yield: 51%. Mp. 160-164°C. Mass (EI), *m*/*z* (*Iᵣₑₗₐₜ*.(%)): 330.1986 [M]⁺ (57). C₁₂H₁₂BrNO₃S.

### e) Preparation of ethyl 2-(acetylamino)-5-bromo-6-methoxy-1-benzothiophene-3-carboxylate 17.

Compound **16** (0.3 g) is suspended in acetic anhydride (5 ml) and boiled for 1 hour. First, a solution is formed, then after 30 minutes of boiling, a precipitate is formed. The reaction mixture is cooled, the precipitate is filtered off and washed with water, ethyl alcohol, and hexane. Yield: 89%. Mp. 215-210°C. Mass (EI), *m*/*z* (*Iᵣₑₗₐₜ.*(%)): 372.2353 [M]⁺ (65). C₁₄H₁₄BrNO₃S.

### f) Preparation of ethyl 2-[acetyl(benzyl)amino]-5-bromo-6-methoxy-1-benzothiophene-3-carboxylate 18.

The synthesis method is similar to that of compounds **3a-s.** The reaction is carried out for 96 hours. Acetone is added to the residue obtained after evaporation of ethyl acetate, and reaction product **18** is filtered off. Yield: 75%. Mp. 175-180°C. Mass (EI), *m*/*z* (*Iᵣₑₗₐₜ.*(%)): 462.3578 [M]⁺ (57). C₂₁H₂₀BrNO₄S.

### Example 49.

g) *Ethyl 2-[acetyl(benzyl)amino]-5-bromo-6-hydroxy-1-benzothiophene-3-carboxylate **19.***

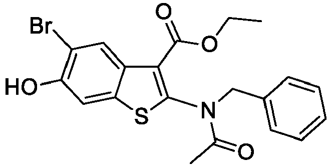

While cooling, boron tribromide (1.3 ml, 10 mmol) is added to a solution of compound **18** (0.64 g, 1 mmol) in 20 ml of chloroform, and the mixture is stirred at room temperature for 1 hour and poured into 60 ml of ice water. The organic layer is separated, chloroform is evaporated, the residue is stirred with water for 30 min. The precipitate is filtered off, washed with water, ethyl alcohol, and hexane, and crystallized from ethanol. Yield: 93%. Mp. 201-203°C. Mass (EI), *m*/*z* (*Iᵣₑₗₐₜ*.(%)): 448.3313 [M]⁺ (38). C₂₀H₁₈BrNO₄S.¹H NMR (300 MHz, DMSO-d₆) δ 1.25 (t, *J* = 7.1 Hz, 3H, CH₃CH₂), 1.95 (s, 3H, CH₃CO), 4.18 (s, 2H, CH₃CH₂), 4.58 (br. s, 1H, NCH₂), 5.06 (br. s, 1H, NCH₂), 7.05 - 7.35 (m, 5H, Ph), 7.45 (s, 1H, H7), 8.41 (s, 1H, H4), 10.71 (s, 1H, OH).

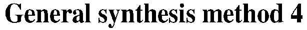

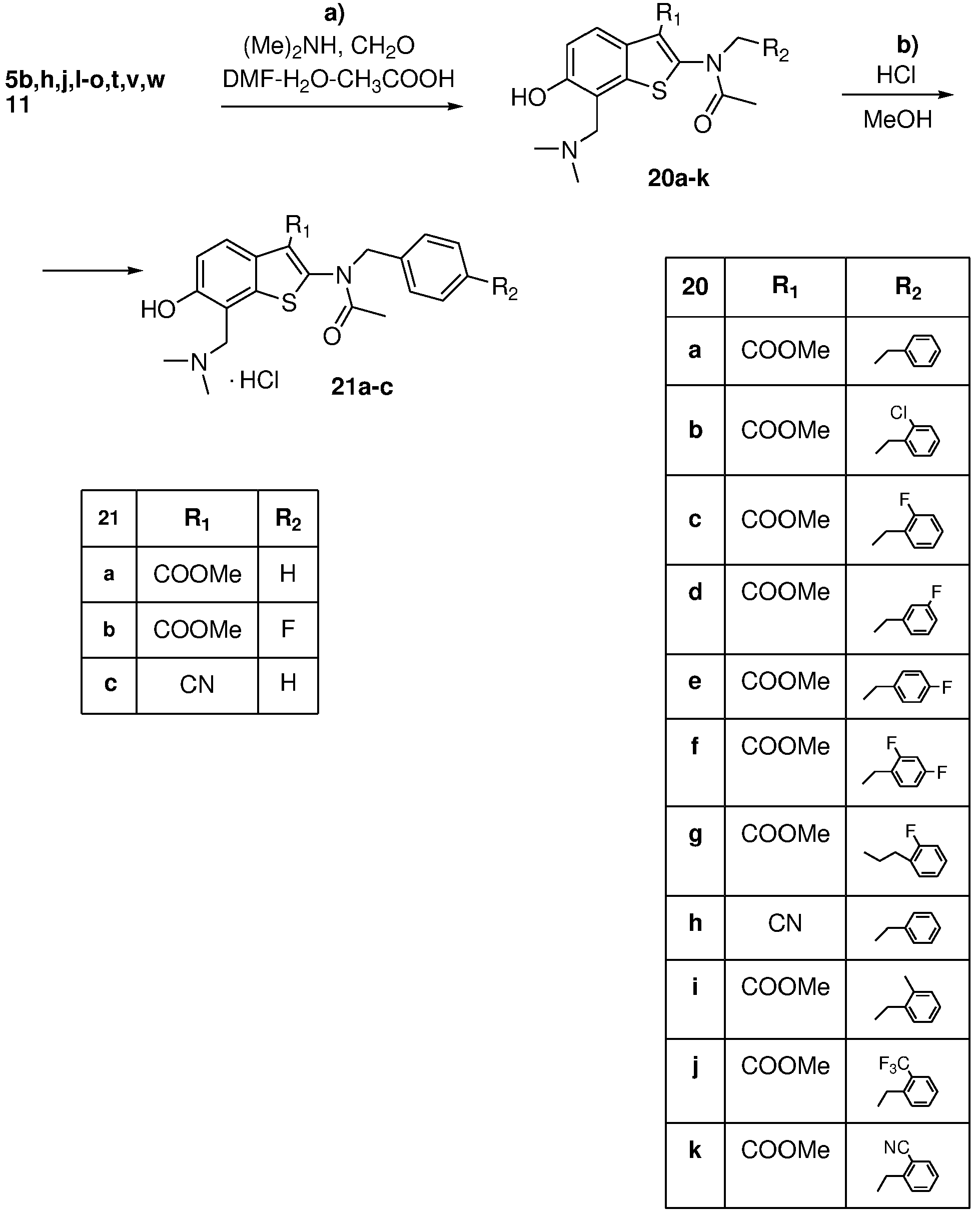

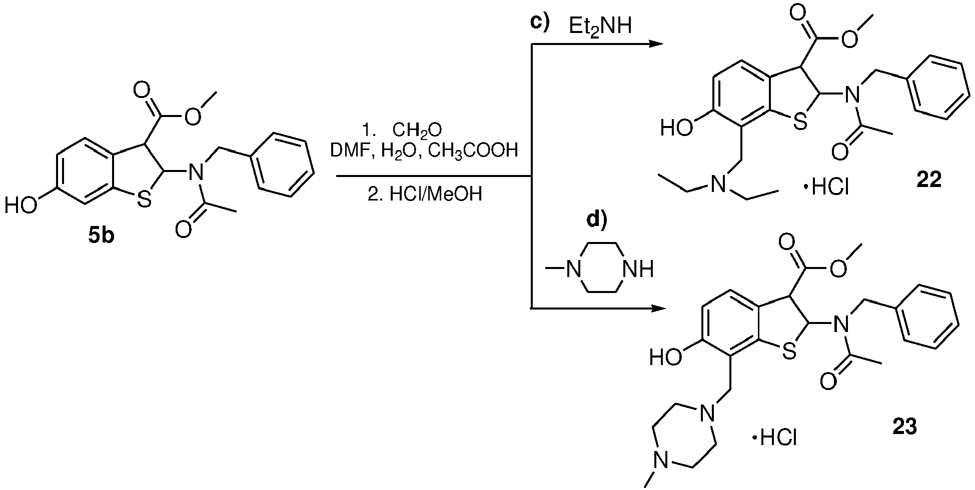

a) Preparation of *methyl 2-[acetyl(R₂,R₃,R₄,R₅-benzyl)amino-7-[(dimethylamino)methyl]-6-hydroxy-1-benzothiophene-3-carboxylate derivatives **20(a-g)*** 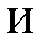 *N-benzyl-N-{3-(cyanocarbonyl)-7-[(dimethylamino)methyl]-6-hydroxy-1-benzothien-2-yl}acetamide derivatives **20h.***

An aqueous solution of 40% dimethylamine (0.563 g, 5.00 mmol), 1 ml of acetic acid and an aqueous solution of 37% formaldehyde (0.216 g, 2.00 mmol ) are added to a solution of compound **5b**, **h, l-o, w** or **11** (1.00 mmol) in 5 ml of DMF. The solution is stirred at room temperature for 24 hours (in case of **20a-g**) or for 72 hours (in case of **20**h). An aqueous solution of sodium carbonate (1.8 g in 7 ml of water) is added to the reaction mixture, stirred for 30 minutes, and transferred to a separating funnel, where 70 ml of ethyl acetate and 30 ml of water are added. The organic layer is washed with an additional 50 ml of water and dried with sodium sulfate. Ethyl acetate is evaporated. Hexane is added to the residue, and the precipitate is filtered off. When synthesizing **20h,** oil is obtained after evaporation of the ethyl acetate.

### Example 50.

*Methyl 2-[acetyl(benzyl)amino-7-[(dimethylamino)methyl]-6-hydroxy-1-benzothiophene-3-carboxylate **20a.***

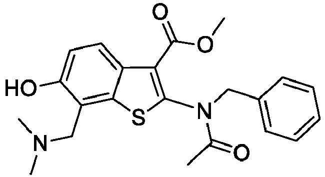

Yield: 64%. Mp. 135-140°C. Mass (EI), *mlz* (*Iᵣₑₗₐₜ.*(*%*)): 412.503 [M]⁺ (79). C₂₂H₂₄N₂O₄S. ¹H NMR (300 MHz, DMSO-d₆) δ 1.94 (s, 3H, CH₃CO), 2.20 (d, *J* = 1.4 Hz, 6H, N(CH₃)₂), 3.66 (s, 2H, CH₂N(CH₃)₂), 3.70 (s, 3H, OCH₃), 4.83 (br. s, 2H, NCH₂), 6.99 (d, *J* = 8.8 Hz, 1H, H5), 7.15 - 7.33 (m, 6H), 7.98 (d, *J=* 8.8 Hz, 1H, H4).

### Example 51.

*Methyl 2-[acetyl(2-chlorobenzyl)amino-7-[(dimethylamino)methyl]-6-hydroxy-1-benzothiophene-3-carboxylate **20b.***

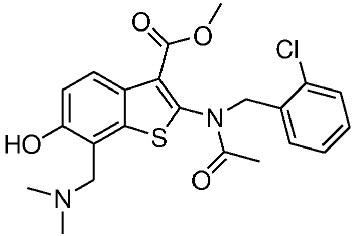

Yield: 82%. Mp. 132-136°C. Mass (EI), *mlz* (*Iᵣₑₗₐₜ.*(*%*)): 446.9478 [M]⁺ (80). C₂₂H₂₃ClN₂O₄S. ¹H NMR (200 MHz, DMSO-d₆) δ 1.92 (s, 3H, CH₃CO), 2.50 (s, 6H, N(CH₃)₂), 3.64 (s, 2H, CH₂N(CH₃)₂), 3.68 (s, 3H, OCH₃), 4.79 (d, *J* = 12.5Hz, 1H, NCH₂Ph), 4.96 (d, *J* = 12.5Hz, 1H, NCH₂Ph), 7.00 (d, *J* = 8.9 Hz, 1H, H5), 7.07 - 7.18 (m, 2H), 7.28 (t, *J* = 7.3 Hz, 2H), 7.98 (d, *J* = 8.9 Hz, 1H, H4).

### Example 52.

*Methyl 2-[acetyl(2-fluorobenzyl)amino-7-[(dimethylamino)methyl]-6-hydroxy-1-benzothiophene-3-carboxylate **20c.***

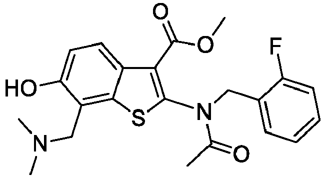

Yield: 78%. Mp. 120-125°C. Mass (EI), *mlz* (*Iᵣₑₗₐₜ.*(*%*)): 430.4935 [M]⁺ (86). C₂₂H₂₃FN₂O₄S. ¹H NMR (200 MHz, DMSO-d₆) δ 1.92 (s, 3H, CH₃CO), 2.40 (s, 6H, N(CH₃)₂), 3.68 (s, 3H, OCH₃), 3.90 (s, 2H, CH₂N(CH₃)₂), 4.85 (d, *J* = 12.5Hz, 1H, NCH₂Ph), 4.97 (d, *J =* 12.5Hz, 1H, NCH₂Ph), 7.02 (d, *J=* 8.9 Hz, 1H, H5), 7.09 - 7.18 (m, 2H), 7.38 (t, *J=* 7.3 Hz, 2H), 8.06 (d, *J* = 8.9 Hz, 1H, H4).

### Example 53.

*Methyl 2-[acetyl(3-fluorobenzyl)amino-7-[(dimethylamino)methyl]-6-hydroxy-1-benzothiophene-3-carboxylate **20d.***

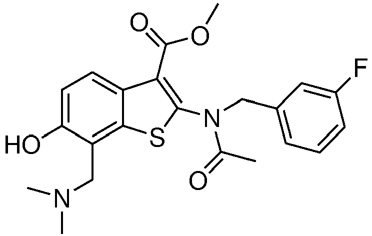

Yield: 80%. Mp. 142-144°C. Mass (EI), *mlz* (*Iᵣₑₗₐₜ.*(*%*)): 430.4935 [M]⁺ (90). C₂₂H₂₃FN₂O₄S. ¹H NMR (300 MHz, DMSO-d₆) δ 1.96 (s, 3H, CH₃CO), 2.45 (s, 6H, N(CH₃)₂), 3.56 (s, 2H, CH₂N(CH₃)₂), 3.74 (s, 3H, OCH₃), 4.68 (s, 1H, NCH₂), 4.99 (s, 1H, NCH₂), 6.98 (dd, *J* = 8.9, 2.5 Hz, 1H), 7.01 - 7.07 (m, 2H), 7.10 (dd, *J* = 8.9, 2.5 Hz, 1H, H5), 7.27 - 7.40 (m, 1H), 8.07 (d, *J* = 8.9 Hz, 1H, H4).

### Example 54.

*Methyl 2-[acetyl(4-fluorobenzyl)amino-7-[(dimethylamino)methyl]-6-hydroxy-1-benzothiophene-3-carboxylate **20e.***

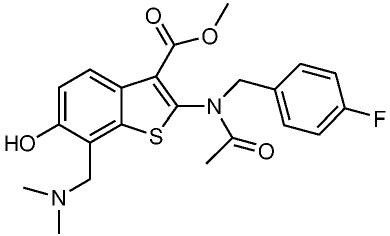

Yield: 46%. Mp. 145-149°C. Mass (EI), *mlz* (*Iᵣₑₗₐₜ.*(*%*)): 430.4935 [M]⁺ (93). C₂₂H₂₃FN₂O₄S. ¹H NMR (200 MHz, DMSO-d₆) δ 1.92 (s, 3H, CH₃CO), 2.38 (s, 6H, N(CH₃)₂), 3.54 (s, 2H, CH₂N(CH₃)₂), 3.72 (s, 3H, OCH₃), 4.80 (d, *J* = 12.5Hz, 1H, NCH₂Ph), 5.02 (d, *J* = 12.5Hz, 1H, NCH₂Ph), 7.05 (d, *J* = 8.9 Hz, 1H, H5), 7.13 - 7.20 (m, 2H), 7.35 (t, *J* = 7.3 Hz, 2H), 8.08 (d, *J* = 8.9 Hz, 1H, H4).

### Example 55.

*Methyl 2-[acetyl(2,4-difluorobenzyl)amino-7-[(dimethylamino)methyl]-6-hydroxy-1-benzothiophene-3-carboxylate **20f**.*

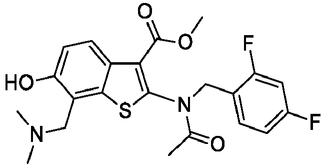

Yield: 44%. Mp. 118-122°C. Mass (EI), *mlz* (*Iᵣₑₗₐₜ.*(%)): 448.484 [M]⁺ (35). C₂₂H₂₃F₂N₂O₄S. ¹H NMR (200 MHz, DMSO-d₆) δ 1.91 (s, 3H, CH₃CO), 2.18 (s, 6H, N(CH₃)₂), 3.65 (s, 2H, CH₂N(CH₃)₂), 3.69 (s, 3H, OCH₃), 4.79 (d, *J* = 13.6 Hz, 1H, NCH₂Ph), 4.91 (d, *J* = 13.6Hz, 2H, NCH₂Ph), 7.00 (m, 1H), 7.01 (d, *J* = 8.8 Hz, 1H, H5), 7.14 (td, *J* = 9.9, 2.6 Hz, 1H), 7.31 (td, *J* = 8.6, 6.6 Hz, 1H, H6'), 7.98 (d, *J* = 8.8 Hz, 1H, H4).

### Example 56.

*Methyl 2-{acetyl[2-(2-fluorophenyl)ethyl]amino}-7-[(dimethylamino)methyl]-6-hydroxy-1-benzothiophene-3-carboxylate 20g.*

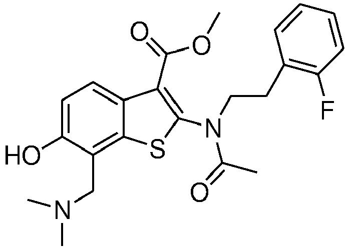

Yield: 67%. Mp. 114-120°C. Mass (EI), *mlz* (*Iᵣₑₗₐₜ.*(*%*)): 444.5201 [M]⁺ (76). C₂₃H₂₅FN₂O₄S. ¹H NMR (200 MHz, DMSO-d₆) δ 1.92 (s, 3H, CH₃CO), 2.60 (s, 6H, N(CH₃)₂), 2.96 (s, 2H, PhCH₂), 3.76 (s, 2H, NCH₂), 3.68 (s, 3H, OCH₃), 3.78 (s, 2H, CH₂N(CH₃)₂), 7.08 (d, *J* = 8.9 Hz, 1H, H5), 7.12 - 7.19 (m, 2H), 7.30 (t, *J* = 7.3 Hz, 2H), 8.02 (d, *J* = 8.9 Hz, 1H, H4).

### Example 57.

*N-benzyl-N-{3-(cyanocarbonyl)-7-[(dimethylamino)methyl]-6-hydroxy-1-benzothien-2-yl)acetamide **20h**.*

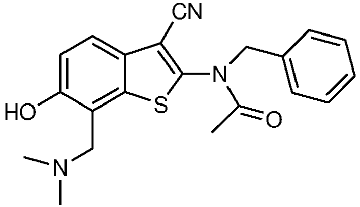

Yield: 89%. Oil. Mass (EI), *mlz* (*Iᵣₑₗₐₜ.*(*%*)): 4074866.484 [M]⁺ (55). C₂₂H₂₁N₃O₃S. ¹H NMR (200 MHz, DMSO-d₆) δ 2.14 (s, 3H, CH₃CO), 2.76 (s, 6H), 4.30 (s, 2H, CH₂N(CH₃)₂), 4.89 (s, 2H, NCH₂Ph), 7.00 - 7.45 (m, 6H), 7.68 (d, *J* = 8.7 Hz, 1H, H4).

b) Preparation of *methyl 2-[acetyl(R₂-benzyl)amino-7-[(dimethylamino)methyl]-6-hydroxy-1-benzothiophene-3-carboxylate hydrochloride derivatives **21a,b*** 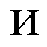 *N-benzyl-N-{3-(cyanocarbonylcarbonyl)-7-[(dimethylamino)methyl]-6-hydroxy-1-benzothiophene-2-yl}acetamide hydrochloride **21c.***

Compounds **20a,e** (1 mmol) and **20h** (1 mmol) are suspended in 15 ml of methanol. a solution of HCl in methanol to pH~2 is added to the suspension. The suspension is stirred at room temperature for 30 min, the alcohol is evaporated off, diethyl ether is added, the precipitate is filtered off and washed thoroughly with ether. If **20h** is used as starting compound, the suspension is stirred at room temperature for 2 h, the precipitate is filtered off and washed with ether.

### Example 58.

*Methyl 2-[acetyl(2-methylbenzyl)amino-7-[(dimethylamino)methyl]-6-hydroxy-1-benzothiophene-3-carboxylate 20i.*

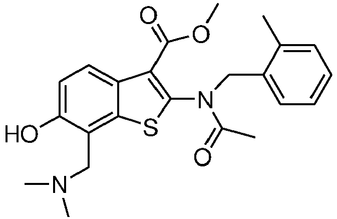

Yield: 76%. Mp. 155-160°C. Mass (EI), *mlz* (Irelat.(%)): 426.5296 M]⁺ (79). C₂₃H₂₆N₂O₄S. ¹H NMR (300 MHz, DMSO-d₆) δ 1.94 (s, 3H, CH₃CO), 2.15 (d, *J* = 1.4 Hz, 6H, N(CH₃)₂), 2.25 (s, 3H, CH₃Ar), 3.65 (s, 2H, CH₂N(CH₃)₂), 3.72 (s, 3H, OCH₃), 4.67 (s, 2H, NCH₂), 6.92 (d, *J* = 8.8 Hz, 1H, H5), 7.14 - 7.35 (m, 5H), 8.10 (d, *J* = 8.8 Hz, 1H, H4).

### Example 59.

*Methyl 2-[acetyl(2-(trifluoromethyl)benzyl)amino-7-[(dimethylamino)methyl]-6-hydroxy-1-benzothiophene-3-carboxylate 20j.*

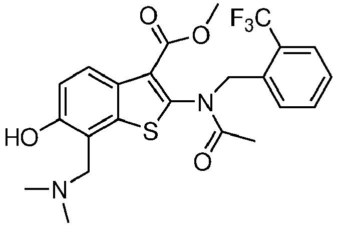

Yield: 46%. Mp. 184-188°C. Mass (EI), *m*/*z* (*Iᵣₑₗₐₜ.*(%)): 480.501 [M]⁺ (67). C₂₃H₂₃F₃N₂O₄S. ¹H NMR (300 MHz, DMSO-d₆) δ 1.98 (s, 3H, CH₃CO), 2.24 (d, *J=* 1.4 Hz, 6H, N(CH₃)₂), 3.63 (s, 3H, OCH₃), 3.89 (s, 2H, CH₂N(CH₃)₂), 4.87 (br. s, 2H, NCH₂), 7.02 (d, *J=* 8.8 Hz, 1H, H5), 7.25 - 7.43 (m, 5H), 8.09 (d, *J* = 8.8 Hz, 1H, H4).

### Example 60.

*Methyl 2-[acetyl(2-cyanobenzyl)amino-7-[(dimethylamino)methyl]-6-hydroxy-1-benzothiophene-3-carboxylate **20k.***

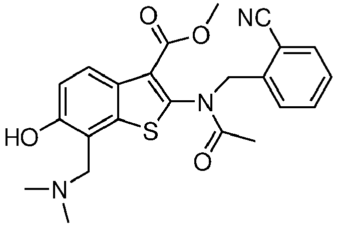

Yield: 73%. Mp. 178-182°C. Mass (EI), *mlz (Irelat.(%)):* 437.5125 [M]⁺ (69). C₂₃H₂₃N₃O₄S. ¹H NMR (300 MHz, DMSO-d₆) δ 1.92 (s, 3H, CH₃CO), 2.26 (d, *J =* 1.4 Hz, 6H, N(CH₃)₂), 3.71 (s, 2H, CH₂N(CH₃)₂), 3.73 (s, 3H, OCH₃), 4.89 (br. s, 2H, NCH₂), 6.97 (d, *J* = 8.8 Hz, 1H, H5), 7.06 - 7.28 (m, 5H), 7.98 (d, *J* = 8.8 Hz, 1H, H4).

### Example 61.

*Methyl 2-[acetyl(benzyl)amino-7-[(dimethylamino)methyl]-6-hydroxy-1-benzothiophene-3-carboxylate hydrochloride **21a**.*

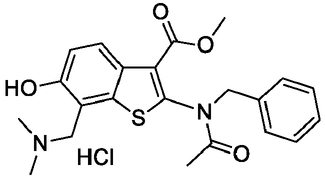

Yield: 95%. Mp. 190-194°C. Mass (EI), *mlz* (*Iᵣₑₗₐₜ.*(*%*)): 448.9637 [M]⁺ (30). C₂₂H₂₅ClN₂O₄S. ¹H NMR (200 MHz, DMSO-d₆) δ 1.97 (s, 3H, CH₃CO), 2.75 (s, 6H), 3.71 (s, 3H, OCH₃), 4.35 (s, 2H, CH₂N(CH₃)₂), 4.72 (d, *J* = 14.8 Hz, 1H, NCH₂), 4.95 (d, *J* = 14.8 Hz, 1H, NCH₂), 7.07 - 7.42 (m, 6H), 8.19 (d, *J=* 8.9 Hz, 1H), 9.92 (s, 1H, H4), 11.20 (s, 1H, OH).

### Example 62.

*Methyl 2-[acetyl(4-fluorobenzyl)amino-7-[(dimethylamino)methyl]-6-hydroxy-1-benzothiophene-3-carboxylate hydrochloride **21b.***

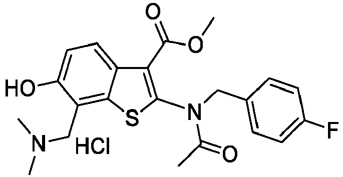

Yield: 78%. Mp. 130°C with decomposition. Mass (EI), *mlz* (*Iᵣₑₗₐₜ.*(*%*)): 466.9541 [M]⁺ (35). C₂₂H₂₄ClFN₂O₄S. ¹H NMR (200 MHz, DMSO-d₆) δ 1.96 (s, 3H, CH₃CO), 2.76 (d, *J* = 4.2 Hz, 6H), 3.72 (s, 3H, OCH₃), 4.36 (d, *J* = 4.7 Hz, 2H CH₂N(CH₃)₂), 4.70 (d, *J* = 14.5Hz, 1H, NCH₂Ph), 4.92 (d, *J* = 14.5Hz, 1H, NCH₂Ph), 6.97 - 7.42 (m, 6H), 8.19 (d, *J* = 8.9 Hz, 1H, H4), 9.94 (br. s, 1H, NH), 11.17 (s, 1H, OH).

### Example 63.

*N-benzyl-N-{3-(cyanocarbonylcarbonyl)-7-[(dimethylamino)methyl]-6-hydroxy-1-benzothien-2-yl)acetamide hydrochloride 21c.*

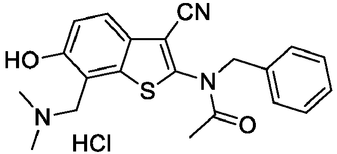

Yield: 58%. Mp. 120-125°C. Mass (EI), *mlz* (*Iᵣₑₗₐₜ.*(*%*)): 407.484 [M]⁺ (55). C₂₂H₂₂ClN₃O₃S.¹H NMR (200 MHz, DMSO-d₆) δ 2.14 (s, 3H, CH₃CO), 2.76 (s, 6H), 4.38 (s, 2H, CH₂N(CH₃)₂), 5.00 (s, 2H, NCH₂Ph), 7.04 - 7.55 (m, 6H), 7.75 (d, *J* = 8.7 Hz, 1H, H4), 10.13 (br. s, 1H, NH), 11.43 (s, 1H, OH).

### Example 64.

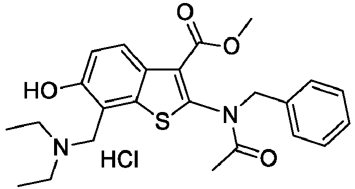

*Methyl 2-[acetyl(benzyl)amino-7-[(dimethylamino)methyl]-6-hydroxy-1-benzothiophene-3-carboxylate hydrochloride **22.***

c) A solution of diethylamine (0.31 g, 4.00 mmol), 0.5 ml of acetic acid, and 37% aqueous formaldehyde solution (0.21 g, 3.00 mmol) are added to a solution of compound **5b** (0.8 mmol) in 10 ml of methanol was added. The solution is stirred at room temperature for 48 hours. An aqueous solution of sodium carbonate (0.7 g in 5 ml of water) is added to the reaction mixture and stirred for 30 minutes. The mixture is transfer to a separating funnel, and 70 ml of ethyl acetate and 30 ml of water are added. The organic layer is washed with an additional 50 ml of water and dried with sodium sulfate. Ethyl acetate is evaporated to provide oil. The oil is dissolved in 5 ml of ethanol, a solution of HCl in methanol is added to pH~2. The solution is stirred at room temperature for 30 min, alcohol is evaporated off, diethyl ether is added, the precipitate is filtered off and washed thoroughly with ether. Yield: 65%. Mp. 210-215°C. Mass (EI), *mlz* (*Iᵣₑₗₐₜ.*(*%*)): 477.0168 [M]⁺ (35). C₂₄H₂₉ClN₂O₄S. ¹H NMR (300 MHz, DMSO-d₆) δ 1.27 (t, *J* = 7.2 Hz, 6H), 1.98 (s, 3H, CH₃CO), 3.11 (br. s, 4H), 3.73 (s, 3H, OCH₃), 4.31 (s, 2H), 4.68 (s, 1H, NCH₂), 4.99 (s, 1H, NCH₂), 7.16 - 7.35 (m, 6H), 8.19 (d, *J* = 8.9 Hz, 1H, H4), 9.42 (br. s, 1H, NH), 11.17 (s, 1H, OH).

### Example 65.

*Methyl 2-[acetyl(benzyl)amino-6-hydroxy-7-[(4-methylpiperazin-1-yl)methyl]-1-benzothiophene-3-carboxylate hydrochloride **23.***

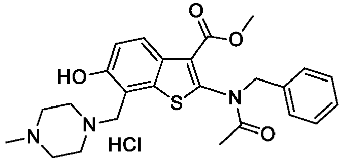

d) A solution of methylpiperazine (0.42 g, 4.00 mmol), 0.5 ml of acetic acid, and 37% aqueous formaldehyde solution (0.21 g, 3.00 mmol) are added to a solution of compound **5b** (0.8 mmol) in 10 ml of methanol. The solution is stirred at room temperature for 15 days. Then the reaction mixture is treated and compound **23** is isolated, similarly to the synthesis of compound **22.**

Yield: 55%. Mp. 170°C with decomposition. Mass (EI), *mlz* (*Iᵣₑₗₐₜ.*(*%*)): 504.0422 [M]⁺ (27). C₂₅H₃₀ClN₃O₄S. ¹H NMR (300 MHz, DMSO-d₆) δ 1.92 (s, 3H, CH₃CO), 2.15 (s, 3H), 2.30 (br. m, 8H), 3.66 (d, *J* = 10.4 Hz, 2H), 3.74 (s, 3H, OCH₃), 4.50 (d, *J* = 14.9 Hz, 1H, NCH₂), 5.13 (d, *J* = 14.9 Hz, 1H, NCH₂), 7.01 (d, *J* = 8.8 Hz, 1H, H5), 7.17 - 7.38 (m, 6H, Ph, NH), 8.00 (d, *J* = 8.8 Hz, 1H, H4).

### General synthesis method 5.

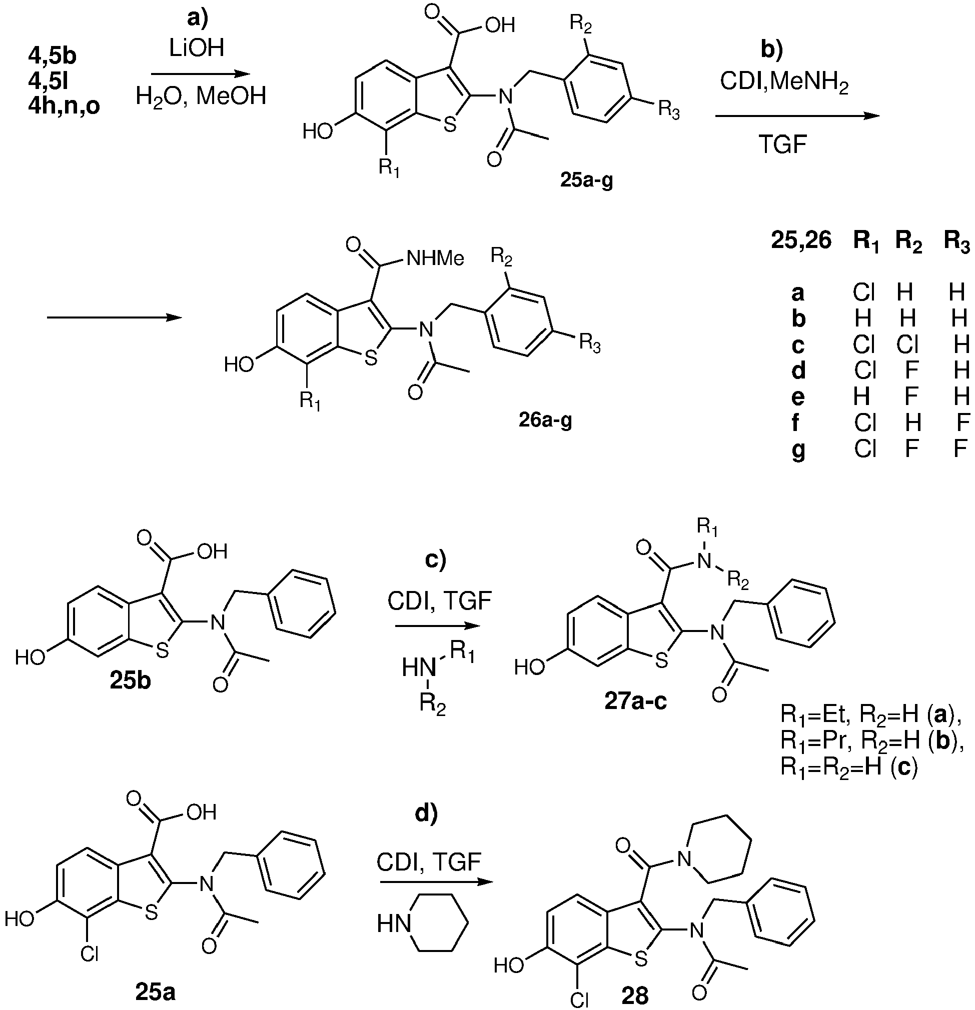

### a) Preparation of 2-[acetyl(R₂,R₃-benzyl)amino]-6-hydroxy-7-R₁-1-benzothiophene-3-carboxylic acid derivatives 25a-g.

A suspension of the appropriate starting material (2 mmol) in aqueous methanol (10 ml of water and 25 ml of methanol) and lithium hydroxide (6 mmol) is heated in a bomb on an oil bath at a bath temperature of 80°C for 20 hours, then cooled, and HCl is added to the solution to pH ~ 2. Alcohol is evaporated, water is added to the oily residue and decanted. Water is added again and stirred with cooling for 3 hours. The precipitate is filtered off, washed with water, and triturated on the filter with a mixture of hexane and diethyl ether (1:1) or only with ether.

### Example 66.

*2-[acetyl(benzyl)amino]-7-chloro-6-hydroxy-1-benzothiophene-3-carboxylic acid **25a**.*

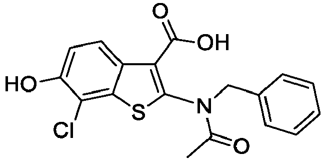

Yield: 80%. Mp. 240-245°C. Mass (EI), *mlz* (*Iᵣₑₗₐₜ.*(*%*)): 375.8268 [M]⁺ (18). C₁₈H₁₄ClNO₄S. ¹H NMR (300 MHz, DMSO-d₆) δ 1.96 (s, 3H, CH₃CO), 4.39 (d, *J* = 15.1 Hz, 1H, NCH₂), 5.31 (d, *J* = 15.1Hz, 1H, NCH₂), 7.17 (d, *J* = 8.9 Hz, 1H, H5), 7.21 - 7.34 (m, 5H, Ph), 8.15 (d, *J* = 8.9 Hz, 1H, H4), 10.63 (s, 1H, OH), 13.34 (s, 1H, COOH).

### Example 67.

*2-[acetyl(benzyl)amino]-6-hydroxy-1-benzothiophene-3-carboxylic acid 25b.*

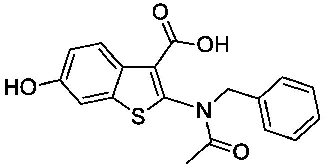

Yield: 86%. Mp. 130-135°C. Mass (EI), *mlz* (*Iᵣₑₗₐₜ.*(*%*)): 341.382 [M]⁺ (96). C₁₈H₁₅NO₄S. ¹H NMR (200 MHz, DMSO-d6) δ 2.01 (s, 3H, CH₃CO), 4.80 (br. s, 2H, NCH₂), 6.91 (dd, *J* = 8.7, 2.4 Hz, 1H, H5), 7.15 - 7.39 (m, 6H), 7.58 (d, *J* = 8.8 Hz, 1H, H4), 9.78 (s, 1H, OH), 12.22 (s, 1H, COOH).

### Example 68.

*2-[acetyl(2-chlorobenzyl)amino]-7-chloro-6-hydroxy-1-benzothiophene-3-carboxylic acid 25c.*

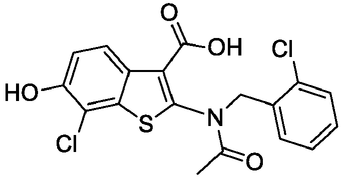

Yield: 73%. Mp. 265-270°C. Mass (EI), *mlz* (*Iᵣₑₗₐₜ.*(*%*)): 410.2716 [M]⁺ (15). C₁₈H₁₃Cl₂NO₄S. ¹H NMR (200 MHz, DMSO-d₆) δ 2.01 (s, 3H, CH₃CO), 4.80 (br. s, 2H, NCH₂Ph), 7.16 (d, *J* = 8.9 Hz, 1H, H5), 7.25 - 7.36 (m, 3H), 7.36 - 7.49 (m, 1H), 8.16 (d, *J* = 8.9 Hz, 1H, H4), 10.69 (s, 1H, OH), 13.53 (s, 1H, COOH).

### Example 69.

*2-[acetyfl2-fluorobenzyl)amino]-7-chloro-6-hydroxy-1-benzothiophene-3-carboxylic acid 25d.*

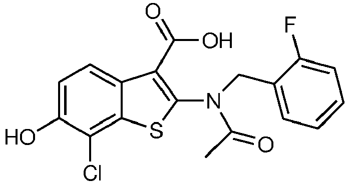

Yield: 63%. Mp. 230-234°C. Mass (EI), *mlz* (Irelat.(%)): 393.8173 [M]⁺ (20). C₁₈H₁₃ClFNO₄S. ¹H NMR (200 MHz, DMSO-d₆) δ 1.95 (s, 3H, CH₃CO), 4.52 (d, 1H, NCH₂Ph), 5.32 (d, 1H, NCH₂Ph), 7.01 - 7.42 (m, 6H), 8.14 (d, *J =* 8.8 Hz, 1H, H4), 10.81 (s, 1H, OH).

### Example 70.

*2-[acetyl(2-fluorobenzyl)amino]-6-hydroxy-1-benzothiophene-3-carboxylic acid **25e.***

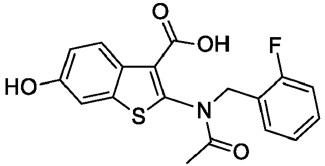

Yield: 85%. %. Mp. 140-145°C. Mass (EI), *mlz* (*Iᵣₑₗₐₜ.*(*%*)): 359.3725 [M]⁺ (17). C₁₈H₁₄FNO₄S. ¹H NMR (200 MHz, DMSO-d₆) δ 2.04 (s, 3H, CH₃CO), 4.82 (br. s, 2H, NCH₂Ph), 7.01 - 7.38 (m, 6H), 7.45 (d, *J* = 8.8 Hz, 1H, H4), 10.01 (s, 1H, OH), 13.42 (s, 1H, COOH).

*2-[acetyl(4-fluorobenzyl)amino]-7-chloro-6-hydroxy-1-benzothiophene-3-carboxylic acid **25f.*** Yield: 83%. Mp. 220-224°C. Mass (EI), *mlz* (*Iᵣₑₗₐₜ.*(*%*)): 393.8173 [M]⁺ (21). C₁₈H₁₃ClFNO₄S.

### Example 71.

*2-[acetyl(2,4-difluorobenzyl)amino]-7-chloro-6-hydroxy-1-benzothiophene-3-carboxylic acid 25g.*

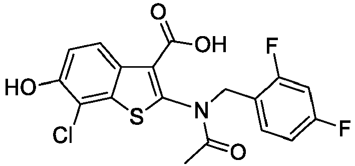

Yield: 68%. Mp. 238-242°C. Mass (EI), *mlz* (*Iᵣₑₗₐₜ.*(*%*)): 411.8077 [M]⁺ (20). C₁₈H₁₂ClF₂NO₄S. ¹H NMR (200 MHz, DMSO-d₆) δ 1.94 (s, 3H, CH₃CO), 4.62 (d, *J* = 14.8 Hz, 1H, NCH₂Ph), 5.12 (d, *J* = 14.8 Hz, 1H, NCH₂Ph), 6.94- 7.24 (m, 2H), 7.19 (d, *J* = 9.0 Hz, 1H, H5), 7.36 (ddd, *J* = 7.0, 8.0, 8.2 Hz, 1H, H6'), 8.14 (d, *J* = 8.9 Hz, 1H, H4), 10.73 (s, 1H, OH).

### b) Preparation of 2-[acetyl(R₂,R₃-benzyl)amino]-7-R₁-6-hydroxy-N-methyl-1-benzothiophene-3-carboxamide derivatives 26a-g.

Carbonyldiimidazole (2 mmol) is added to a solution of appropriate starting compound *25a-g* (1 mmol) in 10 ml of dry THF, and the mixture is stirred at room temperature for 1 hour. An anhydrous solution of methylamine in dioxane (3 mmol) is then added and stirred for 1 hour. Volatiles are removed under vacuum, water is added to the residue and extracted with ethyl acetate. The organic layer is twice washed with water. Ethyl acetate is evaporated, the residue is applied to the column (eluent hexane:acetone 10:4). All fractions with the reaction product, are collected, and solvent is evaporated. The residue is recrystallized from toluene:ethyl acetate (10:1 or 10:4) to provide crystalline compound **26.**

### Example 72.

*2-[acetyl(benzyl)amino]-1-chloro-6-hydroxy-N-methyl-1-benzothiophene-3-carboxamide **26a.***

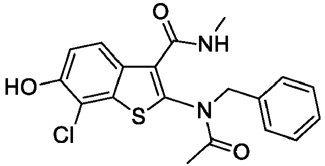

Yield: 53%. Mp. 192-195°C. Mass (EI), *mlz* (*Iᵣₑₗₐₜ.*(*%*)): 388.8687 [M]⁺ (67). C₁₉H₁₇ClN₂O₃S. ¹H NMR (300 MHz, DMSO-d₆) δ 2.05 (s, 3H, CH₃CO), 2.74 (d, *J* = 4.6 Hz, 3H, NCH₃), 4.86 (br. s, 2H, NCH₂), 7.13 (d, *J* = 8.7 Hz, 1H, H5), 7.18 - 7.38 (m, 5H), 7.57 (d, *J* = 8.7 Hz, 1H, H4), 8.09 (br. q, *J* = 4.6 Hz, 1H, NH), 10.59 (s, 1H, OH).

### Example 73.

*2-[acetyl(benzyl)amino)--6-hydroxy-N-methyl-1-benzothiophene-3-carboxamide 26b.*

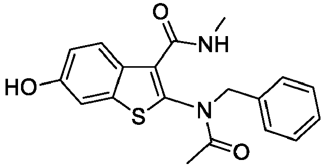

Yield: 33%. Mp. 158-162°C. Mass (EI), *mlz* (*Iᵣₑₗₐₜ*.(%)): 354.4239 [M]⁺ (56). C₁₉H₁₈N₂O₃S. ¹H NMR (200 MHz, DMSO-d₆) δ 2.03 (s, 3H, CH₃CO), 2.74 (d, *J* = 4.5 Hz, 3H, NCH₃), 4.82 (br. s, 2H, NCH₂), 6.92 (dd, *J* = 8.7, 2.4 Hz, 1H, H5), 7.15 - 7.39 (m, 6H), 7.56 (d, *J* = 8.8 Hz, 1H, H4), 8.11 (br. q, *J* = 5.0 Hz, 1H, NH), 9.88 (s, 1H, OH).

### Example 74.

*2-[acetyl(2-chlorobenzyl)amino]-1-chloro-6-hydroxy-N-methyl-1-benzothiophene-3-carboxamide **26c.***

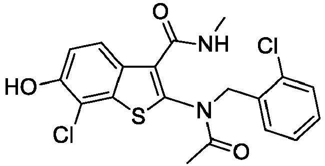

Yield: 53%. Mp. 200-202°C. Mass (EI), *mlz* (*Iᵣₑₗₐₜ.*(*%*)): 423.3134 [M]⁺ (67). C₁₉H₁₆Cl₂N₂O₃S. ¹H NMR (200 MHz, DMSO-d₆) δ 2.08 (s, 3H, CH₃CO), 2.77 (d, *J* = 4.5 Hz, NCH₃), 4.95 (br. s, 2H, NCH₂Ph), 7.14 (d, *J* = 8.7 Hz, 1H, H5), 7.29 - 7.37 (m, 3H), 7.39 - 7.47 (m, 1H), 7.58 (d, *J* = 8.7 Hz, 1H, H4), 8.25 (br. s, 1H, NH), 10.65 (s, 1H, OH).

### Example 75.

*2-[acetyl(2-fluorobenzyl)amino]-7-chloro-6-hydroxy-N-methyl-1-benzothiophene-3-carboxamide **26d.***

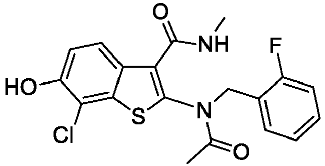

Yield: 38%. Mp. 108-112°C. Mass (EI), *mlz* (*Iᵣₑₗₐₜ.*(*%*)): 406.8591 [M]⁺ (67). C₁₉H₁₆ClFN₂O₃S. ¹H NMR (200 MHz, DMSO-d₆) δ 2.04 (s, 3H, CH₃CO), 2.73 (d, *J* = 4.6 Hz, 3H, NCH₃), 4.91 (br. s, 2H, NCH₂Ph), 7.00 - 7.45 (m, 5H), 7.58 (d, *J=* 8.7 Hz, 1H, H4), 8.21 (br. q, *J* = 4.7 Hz, 1H, NH), 10.66 (s, 1H, OH).

### Example 76.

*2-[acetyl(2-fluorobenzyl)amino]-6-hydroxy-N-methyl-1-benzothiophene-3-carboxamide **26e.***

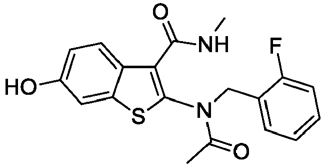

Yield: 35%. Mp. 105-112°C. Mass (EI), *mlz* (*Iᵣₑₗₐₜ.*(*%*)): 372.4144 [M]⁺ (67). C₁₉H₁₇FN₂O₃S. ¹H NMR (200 MHz, DMSO-d₆) δ 2.03 (s, 3H, CH₃CO), 2.74 (d, *J* = 4.6 Hz, 3H, NCH₃), 4.89 (s, 2H, NCH₂Ph), 6.92 (d, *J* = 8.8 Hz, 1H, H5), 7.31 - 7.06 (m, 5H), 7.56 (d, *J* = 8.8 Hz, 1H, H4), 8.15 (br. s, 1H, NH), 9.81 (s, 1H, OH).

### Example 77.

*2-[acetyl(benzyl)amino]-1-chloro-6-hydroxy-N-methyl-1-benzothiophene-3-carboxamide **26f.***

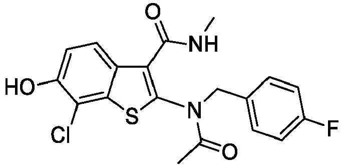

Yield: 53%. Mp. 192-195°C. Mass (EI), *mlz* (*Iᵣₑₗₐₜ.*(*%*)): 406.8591 [M]⁺ (67). C₁₉H₁₆ClFN₂O₃S. ¹H NMR (200 MHz, DMSO-d₆) δ 2.04 (s, 3H, CH₃CO), 2.71 (br. d, *J* = 4.7 Hz, 3H, NCH₃), 4.82 (br. s, 2H, NCH₂Ph), 6.99 - 7.39 (m, 5H), 7.56 (d, *J* = 8.7 Hz, 1H, H4), 8.15 (br. q, *J* = 4.7 Hz, 1H, NH), 10.66 (s, 1H, OH).

### Example 78.

*2-[acetyl(2,4-difluorobenzyl)amino]-7-chloro-6-hydroxy-N-methyl-1-benzothiophene-3-carboxamide **26g.***

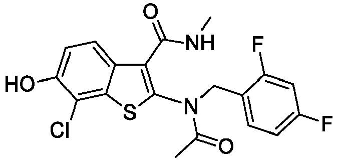

Yield: 38%. Mp. 125-130°C. Mass (EI), *mlz* (*Iᵣₑₗₐₜ.*(*%*)): 424.8496 [M]⁺ (14). C₁₉H₁₅ClF₂N₂O₃S. ¹H NMR (200 MHz, DMSO-d₆) δ 2.04 (s, 3H, CH₃CO), 2.72 (d, *J* = 4.5 Hz, 3H, NCH₃), 4.87 (br. s, 2H, NCH₂Ph), 6.97 - 7.24 (m, 1H), 7.15 (d, *J* = 8.8 Hz, 2H), 7.34 (td, *J* = 8.7, 6.6 Hz, 1H, H6'), 7.57 (d, *J* = 8.7 Hz, 1H, H4), 8.17 (d, *J* = 4.9 Hz, 1H, NH), 10.66 (s, 1H, OH).

### c) Preparation of 2-[acetyl(benzyl)amino]-6-hydroxy-N.N'-R-substituted-1-benzothiophene-3-carboxamide derivatives 27a-d.

The synthesis is similar to the synthesis of the compounds according to examples 69-75, except for the amine used.

### Example 79.

*2-[acetyl(benzyl)amino]-6-hydroxy-N-ethyl-1-benzothiophene-3-carboxamide **27a.***

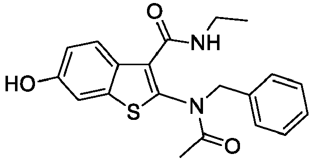

An anhydrous solution of dioxane saturated with ethylamine is used for synthesis.

Yield: 17%. Mp. 138-142°C. Mass (EI), *mlz* (*Iᵣₑₗₐₜ*.(%)): 368.4505 [M]⁺ (38). C₂₀H₂₀N₂O₃S. ¹H NMR (300 MHz, DMSO-d₆) δ 1.11 (t, *J* = 7.2 Hz, 3H, CH₃CH₂), 2.02 (s, 3H, CH₃CO), 3.23 (q, *J* = 7.2 Hz, 2H, CH₃CH₂), 4.91 (br. s, 2H, NCH₂), 6.93 (dd, *J* = 8.8, 2.3 Hz, 1H, H5), 7.08 - 7.45 (m, 6H), 7.56 (d, *J* = 8.8 Hz, 1H, H4), 8.18 (br. s, 1H, NH), 9.77 (s, 1H, OH).

### Example 80.

*2-[acetyl(benzyl)amino]-6-hydroxy-N-propyl-1-benzothiophene-3-carboxamide **27b.***

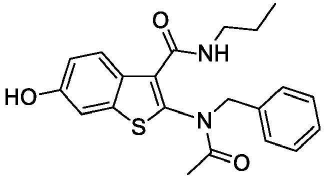

An anhydrous solution of dioxane saturated with propylamine is used for synthesis.

Yield: 12%. Mp. 85-90°C. Mass (EI), *mlz* (*Iᵣₑₗₐₜ.*(*%*)): 382.4771 [M]⁺ (77). C₂₁H₂₂N₂O₃S. ¹H NMR (300 MHz, DMSO-d₆) δ 1.11 (t, *J* = 7.2 Hz, 3H, CH₃CH₂), 2.02 (s, 3H, CH₃CO), 2.21 (m, 2H, CH₃CH₂), 3.23 (q, *J* = 7.2 Hz, 2H, NHCH₂), 4.91 (br. s, 2H, NCH₂), 6.93 (dd, *J* = 8.8, 2.3 Hz, 1H), 7.08 - 7.40 (m, 6H), 7.55 (d, *J* = 8.8 Hz, 1H, H-4), 8.08 (br. t, *J* = 4.3 Hz, 1H, NH), 9.79 (s, 1H, OH).

### Example 81.

*2-[acetyl(benzyl)amino]-6-hydroxy-1-benzothiophene-3-carboxamide 27c.*

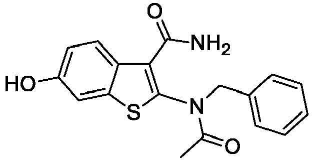

For the synthesis, a methanolic solution of ammonia is used.

Yield: 12%. Mp. 110-115°C. Mass (EI), *mlz* (Irelat.(%)): 340.3973 [M]⁺ (47). C₁₈H₁₆N₂O₃S. ¹H NMR (300 MHz, DMSO-d₆) δ 2.03 (s, 3H, CH₃CO), 4.38 -5.61 (m, 2H, NCH₂), 6.93 (dd, *J* = 8.8, 2.3 Hz, 1H, H5), 7.17 (d, *J* = 2.3 Hz, 1H, H7), 7.21 - 7.37 (m, 5H), 7.65 (d, *J* = 8.8 Hz, 3H, H4, NH₂), 9.74 (s, 1H, OH).

### Example 82.

*N-benzyl-N-[7-chloro-6-hydroxy-3-(piperidin-1-ylcarbonyl)-1-benzothien-2-yl]acetamide* 28.

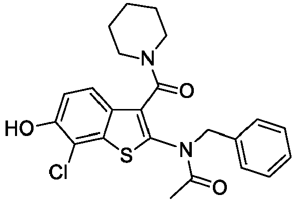

d) Carbonyldiimidazole (1 mmol) is added to a solution of compound **25a** (0.5 mmol) in dry THF (5 ml), and the mixture is stirred at room temperature for 1 hour. Then piperidine (1.5 mmol) is added, and the mixture is further stirred for 18 hours. Volatiles are removed under vacuum, water is added to the residue, and extraction with chloroform is carried out. The organic layer is twice washed with water, chloroform is evaporated, and the residue is applied to a column (eluent hexane:acetone 10:4). Fractions with the reaction product are collected, the solvent is evaporated to provide oil. Hexane and diethyl ether are added in a ratio of 1:1, the precipitate is filtered off. Yield: 12%. Mp. 95-100°C. Mass (EI), *mlz (Irelat.(%)):* 442.9591 [M]⁺ (16). C₂₃H₂₃ClN₂O₃S. ¹H NMR (200 MHz, DMSO-d₆) δ 1.12 - 1.77 (m, 6H, (CH₂)₃), 2.11 (s, 3H, CH₃CO), 3.01 (d, *J* = 12.9 Hz, 2H, NCH₂), 3.92 (d, *J* = 13.0 Hz, 2H, NCH₂), 4.81 (s, 2H, NCH₂Ph), 7.14 (d, *J* = 8.6 Hz, 1H, H5), 7.17 - 7.39 (m, 5H, Ph), 7.43 (d, *J* = 8.6 Hz, 1H, H4).

### General synthesis method 6.

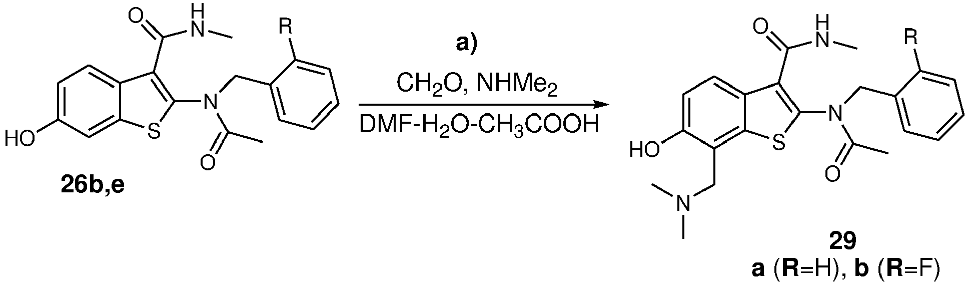

The synthesis is similar to the synthesis of compounds according to examples 69-75.

### Example 83.

*2-[acetyl(benzyl)amino]-7-[(dimethylamino)methyl]-6-hydroxy-N-methyl-1-benzothiophene-3-carboxamide **29a.***

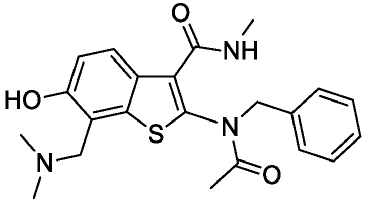

Yield: 18%. Mp. 145-147°C. Mass (EI), *mlz (Irelat.(%)):* 411.5183 [M]⁺ (16). C₂₂H₂₅N₃O₃S. ¹H NMR (200 MHz, DMSO-d₆) δ 2.03 (s, 3H, CH₃CO), 2.19 (s, 6H, N(CH₃)₂), 2.72 (d, *J* = 4.6 Hz, 3H, NCH₃), 3.63 (s, 2H, CH₂N(CH₃)₂), 4.83 (br. s, 2H, NCH₂Ph), 6.94 (d, *J* = 8.7 Hz, 1H, H5), 7.06 - 7.41 (m, 5H), 7.49 (d, *J=* 8.7Hz, 1H, H4), 8.06 (br. q, *J* = 4.6 Hz, 1H, NH).

### Example 84.

*2-[acetyl(2-fluorobenzyl)amino]-7-[(dimethylamino)pnelhyl]-6-hydroxy-N-methyl-1-benzothiophene-3-carboxamide 29b.*

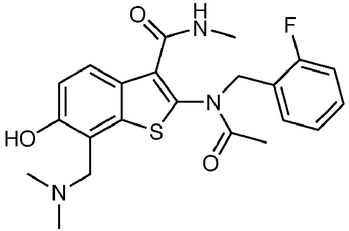

Yield: 65%. Mp. 122-126°C. Mass (EI), *mlz* (*Iᵣₑₗₐₜ.*(*%*)): 429.5988 [M]⁺ (23). C₂₂H₂₄FN₃O₃S. ¹H NMR (200 MHz, DMSO-d₆) δ 1.97 (s, 3H, CH₃CO), 2.20 (s, 6H, N(CH₃)₂), 2.75 (d, *J* = 4.6 Hz, 3H, NCH₃), 3.63 (s, 2H, CH₂N(CH₃)₂), 4.83 (br. s, 2H, NCH₂Ph), 6.99 (d, *J* = 8.7 Hz, 1H, H5), 7.15 - 7.56 (m, 5H), 7.59 (d, *J =* 8.7Hz, 1H, H4), 8.12 (br. q, *J* = 4.6 Hz, 1H, NH).

### Example 85. Description of tests to determine the cytotoxicity and antiviral activity of the compounds of formula I.

### Cytotoxicity of 2-acetamido-6-hydroxy-benzothiophene derivatives

The microtetrazolium test (MTT) was used to study the cytotoxicity of the compounds. For this purpose, a series of threefold dilutions of each compound (300-0.1 µg/ml) was prepared on MEM cell culture medium. MDCK cells were seeded in 96-well plates and incubated for 24 hours at 36°C in 5% CO₂ until a monolayer formed. Serial dilutions of the test substances were added to the wells of the plates (0.2 ml per well) and incubated for 48 h at 36°C in 5% CO₂. The degree of destruction of the cell monolayer was then assessed in the microtetrazolium test (MTT). To this end, the cells were washed twice with physiological phosphate buffer and a solution of 3-(4.5-dimethylthiazolyl-2)-2.5-diphenyltetrazolium bromide (ICN Biochemicals Inc., Aurora, Ohio) (0.5 mg/ml) was added to the wells of the plates in a cell culture medium (0.1 ml per well). After 2 h of incubation, the wells were washed and the stained formazan precipitate was dissolved in DMSO (0.1 ml per well) using a bacterial culture shaker. The optical density in the wells of the plate was then measured on an optical reader ThermoMultiskanFC (ThermoScientific, USA) at a wavelength of 540 nm. Thus obtained absorbance values were expressed as a percentage of control values (cells without test compounds) and, with appropriate concentrations of test compounds, were used for regression analysis using the GraphPadPrism software package (LaJolla, CA). When constructing the regression dependence, a 4-parameter model of a logistic curve with a variable slope was used. Each concentration was tested in duplicate and in triplicate. Based on the data obtained, the 50% cytotoxic dose (CC₅₀) of each compound was calculated (i.e., the concentration of the compound that causes the death of 50% of the cells in culture or a decrease in optical density by half compared to the control wells). CC₅₀ data are given as mean ± SD for triplicates and the results are presented in Table 1.

### Example 86. Determination of activity against the influenza virus of 2-acetamide-6-hydroxy-benzothiophene derivatives of formula I.

The compound at appropriate concentrations was incubated with MDCK cells for 1 h at 36°C. The cell culture was then infected with influenza A/PuertoRico/8/34 (H1N1) (MOI 0.01). The plates were incubated for 24 h at 36°C in the presence of 5% CO₂. 10-fold dilutions were then prepared from the culture medium and used to infect MDCK cells in plates. The plates were incubated for 48 hours at 36°C in the presence of 5% CO₂. The presence of the virus was assessed by the reaction of hemagglutination with chicken erythrocytes. To do this, 100 µl of the culture liquid was transferred into the appropriate wells of round bottom plates for immunological reactions and an equal volume of 1% suspension of chicken erythrocytes in saline was added. The level of virus reproduction in the wells of the panel was assessed by the red blood cell hemagglutination reaction (RHA). The virus titer was taken as the reciprocal of the highest dilution of the virus capable of inducing a positive hemagglutination reaction. The infectious titer of the virus was expressed as 50% of the experimental infectious doses (TCID₅₀) of the virus in 0.2 ml. Each concentration of compounds was tested in duplicate. The antiviral activity of the compounds was assessed by the decrease in virus titer compared to the control. The virus titer was expressed as a percentage of control values (without drugs) and used for regression analysis as described above. Based on the data obtained, a 50% effective dose (IC₅₀) was calculated for each compound, that is, the concentration at which the infectious titer of the virus was reduced by half compared to the control (placebo), and the selectivity index (SI) (the ratio of CC₅₀ to IC₅₀). The calculated IC₅₀ values are given as mean ± SD for three experimental replicates and the results are presented in Table 1.

**Table 1. Influenza virus activity values of the obtained 2-acetamideo-6-hydroxy-benzothiophene derivatives.**

| **Example** | **CC₅₀ (MDCK), µg/ml** | **IC₅₀ (A, H1N1), µg/ml** | **SI*** |
|---|---|---|---|
| **1** | >300 | 6.7±1.1 | 45 |
| **2** | 11.7±0.6 | 0.5±0.7 | 23 |
| **3** | 4.5±0.3 | 0.03±0.003 | 150 |
| **4** | 200±0.0 | 0.34±0.81 | 600 |
| **5** | 4.1±0.2 | 1±0.3 | 4 |
| **7** | 2.2±0.3 | 0.3±0.5 | 7 |
| **8** | 13.9±1.1 | 0.3±0.4 | 46 |
| **9** | 8.3±0.3 | 0.08±0.003 | 104 |
| **16** | 21±1.8 | <3 | 7 |
| **21** | 5.6±0.2 | 0.5±0.9 | 11 |
| **22** | 3.2±0.8 | 0.8±1.2 | 4 |
| **23** | >300 | 4±0.6 | 75 |
| **24** | >300 | 31±2 | 10 |
| **25** | 25.5±2.7 | 2.5±0.3 | 10 |
| **27** | 39.8±2.5 | 3.98±0.4 | 10 |
| **28** | 240±19 | 5.9±0.7 | 41 |
| **29** | >300 | 19±2.1 | 16 |
| **30** | >300 | 30±2.8 | 10 |
| **32** | >300 | 68±8 | 4 |
| **33** | 3.7±0.4 | 1±0.2 | 4 |
| **36** | >300 | 4.9±0.6 | 61 |
| **37** | 24.5±2.2 | 4.9±0.6 | 5 |
| **38** | 30±3.2 | 7.9±1.0 | 4 |
| **41** | 5±0.5 | 1±0.2 | 5 |
| **42** | 10±0.9 | 3±0.4 | 3 |
| **47** | 16.8±1.5 | 0.6±0.7 | 28 |
| **50** | 182±33.5 | 0.6±0.6 | 303 |
| **51** | >300 | 1±0.6 | 300 |
| **52** | 180±16 | 2.2±0.3 | 82 |
| **53** | >300 | 3±0.7 | 100 |
| **55** | >300 | 36±4.1 | 8 |
| **56** | >300 | 3.3±0.4 | 91 |
| **58** | >300 | 19±2.2 | 16 |
| **59** | >300 | 1±0.5 | 300 |
| **60** | >300 | 4±0.3 | 75 |
| **61** | >300 | 1.1±0.2 | 273 |
| **62** | 92±7 | 11.2±1.3 | 8 |
| **63** | 61.5±4.9 | 20.7±3.2 | 3 |
| **64** | 148±11 | 25.3±3.6 | 6 |
| **65** | 18.7±1.6 | 5.9±0.6 | 3 |
| **66** | >300 | 5.3±0.9 | 57 |
| **67** | >300 | 160±19 | 2 |
| **68** | 65±4 | 23±3 | 3 |
| **71** | 210±16 | 26±4 | 8 |
| **72** | 160±11 | 0.1±0.1 | 1600 |
| **73** | >300 | 0.4±0.1 | 750 |
| **74** | 32±4 | 3.1±0.5 | 10 |
| **75** | 28±4 | >10 | 3 |
| **76** | >300 | 18.6±2.8 | 16 |
| **77** | 32.2±2.4 | 2±0.3 | 16 |
| **78** | 68±5 | 5.1±0.6 | 13 |
| **79** | 250±21 | 7.9±0.6 | 32 |
| **80** | 240±16 | 3.1±0.4 | 77 |
| **81** | 251±19 | 44±5 | 6 |
| **82** | 16.3±0.8 | 5.1±0.6 | 3 |
| **84** | >300 | 0.7±0.5 | 428 |
| **Umifenovir** | 16±0.9 | 6.7±2.2 | 2 |
| **Methylthionitrooxodihydrotriazolotriazinide sodium (Triazavirin)** | 298±22 | 202±31 | 1 |
| **Pentandioic acid imidazolylethanamide (Ingavirin)** | 300±12 | 300±14 | 1 |

| | | | |
|---|---|---|---|
| * SI is selectivity index | | | |

### Example 87. Efficacy study of 2-acetamido-6-hydroxy-benzothiophene derivatives in an in vivo model of viral infection

White outbred mice (females) weighing 16-18 g (age 5-6 weeks) were obtained from the Rappolovo nursery in Leningrad region. The information about the experimental group was placed on the front surface of the cages and included the start date of the experiment, the date of infection, the name and mode of administration of the study drug. The animals were kept under standard conditions as prescribed by the Chief State Sanitary Doctor of the Russian Federation in the Directive on approval of SP 2.2.1.3218-14 "Sanitary and epidemiological requirements for the arrangement, equipment and maintenance of experimental biological clinics (vivaria)" under No. 51 of 29.08.2014. During the acclimatization period (7 days) and the experiment, the mice were housed in polycarbonate cages (BENEX a.s., Czech Republic, type T3A, S = 1200 cm²) of an open-type barrier in groups of 15 animals, on sawdust bedding. The cages were covered with steel lattice covers with a feeding recess. The floor area in the holding cage for one animal was 80 cm² (minimum allowable area is 40 cm²). The feed for keeping laboratory animals under recipe No. PK-120-2_173 of Laboratorkorm LLC (Moscow) was prepared according to GOST R 50258-92 in accordance with the standards approved by the USSR Ministry of Health (Directive No. 755 of 12.08.1977) and given ad libitum in the feeding recess of the steel lattice cover of the cage. The animals received water purified and normalized with respect to organoleptic properties, in terms of pH, dry residue, reducing substances, carbon dioxide, nitrates and nitrites, ammonia, chlorides, sulfates, calcium and heavy metals on the basis of GOST 51232-98 "Drinking water. General requirements for the organization and methods of quality control. Water in standard drinking bowls with steel spouts was given ad libitum. Wood pellets (Biosfera LLC, St. Petersburg, Russia) were used as bedding.

Test samples: 6 samples of 2-acetamide-6-hydroxy-benzothiophene derivatives (examples **3, 23, 52, 61, 72, 73**). Reference drugs: Umifenovir, Oseltamivir and pentanedioic acid imidazolylethanamide. Before the experiment, the samples were dissolved or suspended in distilled water. The doses of the studied samples were calculated in relative weight units: mg/kg of animal body weight per day.

The experiment involved 100 outbred mature female mice weighing 16-18 g (age 5-6 weeks) and influenza virus A/PuertoRico/8/34 (H1N1) virus from the collection of viral strains of Pasteur Research Institute of Epidemiology and Microbiology (St. Petersburg). Animals were infected intranasally under light ether anesthesia using an infecting dose of 2.5×103 EID₅₀/animal at the rate of 0.025 ml of virus-containing material in each nostril.

The test drugs were administered in daily doses 1 time per day in a volume of 0.2 ml. The compounds were administered orally using a disposable insulin syringe with a gastric tube. The test samples were administered to animals according to the therapeutic and prophylactic scheme: 4 hours before infection, 4 hours after infection, then 1 time per day for 5 days. In total, 24 groups of animals were formed for the experiment.

The death of animals in the experimental groups was recorded daily for 15 days after infection. The assessed survival rates (mortality (M) and protection index (PI)) in the groups were used as the efficacy indicators in the experiment.

A comparative efficacy study of the chemical compounds was carried out in accordance with the Guidelines for the study of the specific antiviral activity of pharmacological substances (2005), Directive "On approval of the rules of laboratory practice in the Russian Federation" under No. 708n of 23.08.2010 by the RF Ministry of Health, and also taking into account the recommendations of international licensing authorities (World Health Organization, Center for Drug Evaluation and Research of the Food and Drug Administration of the US Department of Health and Human Services).

The blind experiment involved the blanked samples, including the control.

The results of studying the dynamics of death of animals in the control and experimental groups are presented in Table 2.

**Table 2. Indicators of the protective activity of 2-acetamido-6-hydroxy-benzothiophene derivatives of formula I in a model of experimental lethal influenza pneumonia in mice.**

| Groups | | Survival rate | | Mortality by days | | | | | | | | | | | | | | Virus titer, lgTCID₅₀ /0.1 ml * |
|---|---|---|---|---|---|---|---|---|---|---|---|---|---|---|---|---|---|---|
| Example | Dose µg/kg/day | Total animals | Mortality, % | | | | | | | | | | | | | | | |
| 23 | 20 | 3/10 | 70 | | | | | | 3 | | 3 | | 1 | | | | | 4.25 |
| | 30 | 5/10 | 50 | | | | | | 1 | | 2 | | 2 | | | | | 3.33 |
| | 60 | 4/10 | 60 | | | | | | 1 | | 2 | | 3 | | | | 1 | 3.33 |
| 73 | 20 | 3/10 | 70 | | | | | | 2 | | | | 3 | | | | 1 | 4.25; |
| | 30 | 5/10 | 50 | | | | | | 2 | | | | 2 | | 1 | | | 3.33 |
| | 60 | 7/10 | 30 | | | | | | | | 2 | | 1 | | | | | 3.1 |
| 61 | 30 | 4/10 | 60 | | | | | | 1 | | | 5 | | | | | | 4.3 |
| | 60 | 8/10 | 20 | | | | | | | | 2 | | | | | | | 2.3 |
| | 90 | 5/10 | 50 | | | | | | 1 | | 1 | | 2 | | 1 | | | 2.1 |
| 52 | 60 | 6/10 | 40 | | | | | | | | 1 | 3 | | | | | | 4.25 |
| | 90 | 9/10 | 10 | | | | | | | | 1 | | | | | | | 2.7 |
| | 120 | 5110 | 50 | | | | | | | | | | 1 | | 4 | | | 2.3 |
| 3 | 30 | 2/10 | 80 | | | | | 3 | | | 1 | | 4 | | | | | 4.5 |
| | 60 | 4/10 | 60 | | | | | | 1 | 3 | | | 2 | | | | | 3.3 |
| | 90 | 4/10 | 60 | | | | | | 2 | | 2 | | 1 | | | | | 3.3 |
| 72 | 20 | 4/10 | 60 | | | | | | 1 | | 2 | 3 | | | | | | 4.0 |
| | 30 | 6/10 | 40 | | | | | | 1 | | 2 | | 1 | | | | | 3 |
| | 60 | 4/10 | 60 | | | | | | | 3 | | | 2 | | | | 1 | 3 |
| Umifenovir | 30 | 4/10 | 60 | | | | | | 2 | | | | 2 | | 2 | | | 4.75 |
| | 60 | 6/10 | 40 | | | | | | | 3 | | | | | 1 | | | 3 |
| | 90 | 7/10 | 30 | | | | | | 1 | | 1 | | | | 1 | | | 2.7 |
| Oseltamivir | 20 | 4/10 | 60 | | | | | | 1 | | 2 | | 2 | | 1 | | | 2.5 |
| Ingavirin * | 90 | 1/10 | 80 | | | | | | 4 | 3 | | | | | 2 | | | 4.75 |
| Control | Saline solution | 0/10 | 100 | | | | | | 2 | 5 | | | 2 | | 1 | | | 6.1 |

| | | | | | | | | | | | | | | | | | | |
|---|---|---|---|---|---|---|---|---|---|---|---|---|---|---|---|---|---|---|
| * Pentandioic acid imidazolylethanamide | | | | | | | | | | | | | | | | | | |

In the group of animals treated with saline solution (control substance), infection with the influenza virus led to specific mortality of animals, starting from the first day after infection. By day 15 of the experiment, the death of animals in the group of animals treated with placebo was 100%. The use of the reference drugs (Umifenovir, Oseltamivir, and pentanedioic acid imidazolylethanamide) led to a decrease in mortality (protection index up to 33%, p<0.0001). The use of the studied compounds affected the animal mortality to varying degrees. The compounds of examples 52, 61 and 73 showed the most outstanding performance in the experiment.

### Example 88. Determination of activity of 2-acetamido-6-hydroxy-benzothiophene derivatives of formula I against SARS-CoV-2 coronavirus on U2-OS ACE2 cells.

The experiment was carried out in accordance with the description in the article by Buchrieser, J. et al. // EMBOJ. 2020, e106267 (doi: 10.15252/embj.2020106267). Thus, U2-OS Ace2 (osteosarcoma cell lines, angiotensin-converting enzyme 2) GFP (glomerular permeability factor) 1-10 and 1-11 (S-Fuse cells) were separated the day before infection as described. The experimental substances were previously dissolved in water at a concentration of 25 µM. 100 µl of experimental compound solution was added to U2-OS ACE2 cells in growth medium (8×10³ cells per well) (10% DMEM (Dulbecco's Minimal Essential Needle Medium) FCS (fetal bovine serum) 1% PS (penicillin-streptomycin)) and incubated for 45-60 min at 37°C. Then 10 µl of virus was added per well (final MOI 0.1). Cells were incubated at 37°C for 20 hours, fixed with 8% PFA for 30 minutes at room temperature and washed with PBS. To stain the cell nuclei and measure their viability, 100 µl of Hoechst solution (Invitrogen Hoechst 33342 nucleic acid stain) was added. The plates are read using an automatic confocal microscope (Opera Phoenix) that measures the number of infected cells (GFP signal) and survival (Hoechst signal). Two experiments were carried out. The stock sample concentration for all samples was 25 mM. The results are presented in Table. 3. IC₅₀ indicates the concentration that inhibits infection by 50%.

**Table 3. Antiviral activity of 2-acetamido-6-hydroxy-benzothiophene derivatives of formula I against the SARS-Cov-2 virus.**

| Example | | IC₅₀, µM | Cytotoxicity µM |
|---|---|---|---|
| **4** | 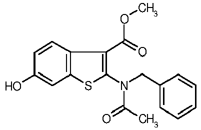 | 10.58 | Non-toxic up to 2500 µM |
| **50** | 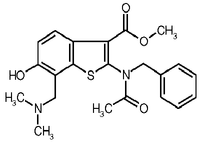 | 9.08 | Non-toxic up to 2500 µM |
| **55** | 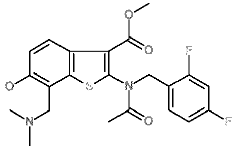 | 15.57 | Non-toxic up to 2500 µM |
| **62** | 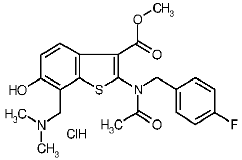 | 11.32 | Non-toxic up to 2500 µM |
| **63** | 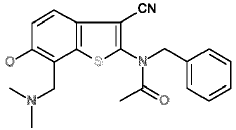 | 40.48 | Non-toxic up to 2500 µM |
| **64** | 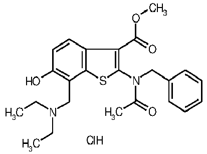 | 9.12 | Non-toxic up to 2500 µM |
| **65** | 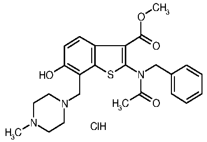 | 9.80 | Non-toxic up to 2500 µM |
| **73** | 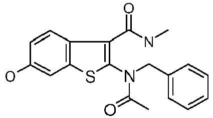 | 23 | Non-toxic up to 2500 µM |
| Umifenovir | | 76 | Non-toxic up to 2500 µM |

### Example 89. Study of acute and subacute toxicity of 2-acetamido-6-hydroxy-benzothiophene derivatives in mice.

The study was carried out on mature mice of the BALB/c line, aged 8-12 weeks, weighing 20 ± 2 g. The acute toxicity of five 2-acetamido-6-hydroxy-benzothiophene derivatives was assessed by the fixed dose method according to OECD Test Guideline 420: "Acute Oral Toxicity - Fixed Dose Procedure" (see data from the OECD online library, URL: https://www.oecd-ilibrary.org/environment/test-no-420-acute-oral-toxicity-fixed-dose-procedure_9789264070943-en) when administered intragastrically to female mice. The acute toxicity was assessed in the preliminary experiment (one animal at the maximum dose) and in the main experiment. Further, the subacute 14-day toxicity was assessed with a 14-day delayed observation period and an assessment of local irritant action when administered intragastrically to mice of both genders at various doses.

The studied substances were administered in fixed volumes in the range of tested doses. The volume administered to mice was 0.1 ml/10 g of body weight. The substances were administered as a suspension in 1% starch paste. Before dosing, animals were restricted in food for at least 3 hours before dosing, water was provided ad libidum.

**Table 4. Acute toxicity of 2-acetamido-6-hydroxy-benzothiophene derivatives in mice.**

| Example | Dose, mg/kg | Died | Survived | GHS hazard class* |
|---|---|---|---|---|
| **23** | 2000 | 0 | 4 | 5 |
| **52** | 2000 | 0 | 4 | 5 |
| **61** | 2000 | 1 | 1 | 4 |
| | 300 | 0 | 4 | |
| **72** | 2000 | 0 | 4 | 5 |
| **73** | 2000 | 0 | 4 | 5 |

| | | | | |
|---|---|---|---|---|
| *Hazard class according to UN Recommendations ST/SG/AC.10/30/Rev.4* "Globally Harmonized System (GHS) of Hazard Classification and Labeling of Chemicals". | | | | |

In the study of subacute toxicity and local irritant action, mature BALB/c mice of both genders were subjected to daily intragastric administration of the test substances indicated in Table 4 for a long time for 14 days at doses of 50, 100 and 200 mg/kg, as well as 1% starch paste in quality control. Each test group consisted of 12 animals (6 females and 6 males), the total number of animals was 192.

It was found that intragastric administration of the studied substances in the studied doses for 14 days did not lead to a statistically significant change in body weight, feed and water intake compared with the control group of animals. Furthermore, there was no change in the general condition, quantitative indicators of blood and functions of the internal organs of experimental mice compared with the control group of animals. The mice, which were treated intragastrically with the test substances at the indicated doses, demonstrated no changes in locomotor and exploratory activity. The histological examination of the internal organs also showed no signs of general toxic and local irritating effects caused by 14-day intragastric administration of the test substances. No pathological changes were observed in animals even 14 days after the cessation of the administration of substances.

## Claims

1. A 2-acetamido-6-hydroxy-benzothiophene derivative of general structural formula I or a pharmaceutically acceptable salt thereof 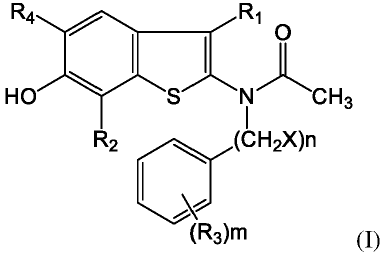 where
R¹ is CN, COOH, COOAlk, CONHAlk, CON(Alk)₂, CONH₂;
R² is H, Hal, CH₂(NAlk)₂;
R³ is Hal, Alk, OAlk, CF₃, or two adjacent R³ groups form -CH=CH-CH=CH- group;
R⁴ is H or Hal;
n = 1-4; m = 1-3;
X is hydrogen or methyl;
Hal is fluorine, chlorine or bromine;
Alk is a linear or branched alkyl group having 1 to 4 carbon atoms, or
in N(Alk)₂ group, two Alk groups together with the nitrogen atom to which they are attached form a -(CH₂)ₚ group, wherein p = 3-5, in which one of said CH₂ groups can be substituted by a nitrogen or oxygen atom, or by a -N-CH₃ group.

2. The 2-acetamido-6-hydroxy-benzothiophene derivative according to claim 1, **characterized in that** R¹ is COOAlk or CONHAlk, n is 1.

3. The 2-acetamido-6-hydroxy-benzothiophene derivative according to claim 1, **characterized in that** R¹ is COOMe, R² is H, Cl or CH₂(NAlk)₂, and n is 1.

4. The 2-acetamido-6-hydroxy-benzothiophene derivative of general formula I according to claim 1, **characterized in that** R¹ is CONHMe, R² is H, Cl or CH₂(NAlk)₂, and n is 1.

5. The 2-acetamido-6-hydroxy-benzothiophene derivative of general formula I according to claim 1, **characterized in that** R¹ is CN, R² is H, Cl or CH₂(NAlk)₂, and n is 1.

6. The 2-acetamido-6-hydroxybenzothiophene derivative of general formula I or a pharmaceutically acceptable salt thereof according to any one of claims 1 to 5, having antiviral activity.

7. The 2-acetamido-6-hydroxy-benzothiophene derivative according to claim 6, **characterized in that** the antiviral activity is antiviral activity against influenza A virus and SARS-CoV-2 coronavirus.

8. Use of the 2-acetamido-6-hydroxy-benzothiophene derivative of general formula I or a pharmaceutically acceptable salt thereof according to any one of claims 1 to 7 as an antiviral component in a drug for the prevention or treatment of a disease mediated by a viral infection.

9. Use of the 2-acetamido-6-hydroxy-benzothiophene derivative according to claim 8, **characterized in that** the viral infection is an infection caused by RNA-containing respiratory viruses, influenza virus, or coronaviruses.

10. Use of the 2-acetamido-6-hydroxy-benzothiophene derivative according to claim 8 or 9, **characterized in that** the viral infection is influenza A virus or SARS-CoV-2 coronavirus, and the disease is influenza or COVID 19.

11. A pharmaceutical composition having antiviral activity, comprising an effective amount of the 2-acetamido-6-hydroxy-benzothiophene derivative according to any one of claims 1 to 7 and at least one pharmaceutically acceptable excipient.

12. The pharmaceutical composition according to claim 11, **characterized in that** said composition is in a suitable form for oral administration or in a suitable form for topical application, or in a suitable form for the preparation of injectable solutions.

13. A method for the prevention or treatment of a disease mediated by a viral infection, comprising administration or application to a subject of an effective amount of the 2-acetamido-6-hydroxy-benzothiophene derivative of general structural formula I or the pharmaceutically acceptable salt thereof according to any one of claims 1 to 7 or of the pharmaceutical composition according to any of claims 11 and 12.

14. The method for the prevention or treatment according to claim 13, **characterized in that** the viral infection is an influenza virus, and the disease is influenza A.
